# EUROPÄISCHE PATENTSCHRIFT

(11) **EP 1 071 409 B1**
(45) Veröffentlichungstag und Bekanntmachung des Hinweises auf die Patenterteilung: **15.10.2003**
(21) Anmeldenummer: 99919226.3
(22) Anmeldetag: 13.04.1999
(51) Int. Cl.: A61K 31/00, A61P 31/00

(54) **VERWENDUNG VON PHOSPHORORGANISCHEN VERBINDUNGEN ZUR THERAPEUTISCHEN UND PROPHYLAKTISCHEN BEHANDLUNG VON INFEKTIONEN**
USE OF ORGANOPHOSPHORIC COMPOUNDS FOR THE THERAPEUTIC AND PREVENTATIVE TREATMENT OF INFECTIONS
UTILISATION DE LIAISONS ORGANOPHOSPHOREES DANS LE TRAITEMENT ET LA PREVENTION D'INFECTIONS

(30) Priorität: 14.04.1998 DE 19816196; 09.06.1998 DE 19825585; 22.09.1998 DE 19843222; 22.09.1998 DE 19843223
(43) Veröffentlichungstag der Anmeldung: 31.01.2001
(73) Patentinhaber: BioAgency AG, 22525 Hamburg (DE)
(72) Erfinder: Hassan, Jomaa, 35392 Giessen (DE)
(74) Vertreter: Dreiss, Fuhlendorf, Steimle & Becker
(86) Internationale Anmeldenummer: EP9902462
(87) Internationale Veröffentlichungsnummer: WO99052515

(56) Entgegenhaltungen:
- EP-A- 0 009 686
- EP-A- 0 256 785
- DE-A- 2 733 658
- JP-A- 57 046 917
- US-A- 4 206 156
- US-A- 4 268 503
- US-A- 4 330 529
- US-A- 4 693 742
- D. GREENWOOD: "Fosfomycin and fosmidomycin" ANTIBIOT. CHEMOTHER. (7TH ED.),1997, Seiten 357-359, XP002113259
- H.C. NEU ET AL.: "In Vitro and In Vivo Antibacterial Activity of FR-31564, a Phosphonic Acid Antimicrobial Agent" ANTIMICROB. AGENTS CHEMOTHER., Bd. 19, Nr. 6, 1981, Seiten 1013-1023, XP002113260
- H.C.NEU ET AL.: "Synergy of Fosmidomycin (FR-31564) and Other Antimicrobial Agents" ANTIMICROB. AGENTS CHEMOTHER., Bd. 22, Nr. 4, 1982, Seiten 560-563, XP002113261
- D. GREENWOOD: "Fosfomycin Trometamol: Activity In Vitro against Urinary Tract Pathogens" INFECTION, Bd. 18, Nr. Suppl. 2, 1990, Seiten S60-S64, XP002113262

## Beschreibung

Die Erfindung betrifft die Verwendung von phosphororganischen Verbindungen und ihren Salzen, Estern und Amiden zur therapeutischen und prophylaktischen Behandlung von Infektionen bei Mensch und Tier, die durch Viren, Pilze und Parasiten hervorgerufen werden. Erfindungsgemäß umfassen die phosphororganischen Verbindungen Phosphonsäurederivate, Phosphinoylderivate und Phosphinsäurederivate.

Die Eignung von Aminohydrocarbylphosphonsäurederivaten sowie einigen ihrer Ester und Salze in Arzneimitteln ist bereits bekannt. Es ist jedoch bisher ausschließlich ihre antimikrobielle Wirksamkeit gegen Bakterien bei Mensch und Tier und gegen Pilze bei Pflanzen beschrieben worden (DE 27 33 658 A1, US 4 143 135, US 4 182 758 und US 4 206 156, US 4 994 447, US 4 888 330, US 4 210 635, US 3 955 958, US 4 196 193, US 4 268 503, US 4 330 529, US 5 189 030, US 3 764 677, US 3 764 676). Weiter sind Substanzen dieser Gruppe als Herbizide (US 4 693 742, US 5 002 602, US 4 131 448, US 3 977 860, US 4 062 669), als Algaezide (US 3 887 353), als das Pflanzenwachstum regulierende Mittel (US 4 127 401, US 4 120 688, US 3 961 934, US 4 431 438, US 3 853 530, US 4 205 977, US 4 025 332, US 3 894 861) und als Reagentien der Farbstoffproduktion (US 4 051 175) beschrieben worden. In der De-27 33 658 A1 ist die Verwendung von Aminohydrocarbylphosphonsäurederivaten zur Behandlung von bakteriellen Erkrankungen beschrieben worden. Zwar spricht das Dokument in der Beschreibungseinleitung von einer mikrobiellen Wirksamkeit gegenüber pathogenen Mikroorganismen, aus dem Gesamtzusammenhang wird jedoch deutlich, daß sich die Erfindung ausschließlich auf Bakterien bezieht. So wird auf Seite 16, 2. Absatz "antimikrobielle Wirksamkeit" als antibakterielle Wirksamkeit" definiert.

Es besteht ein starker Bedarf, für die Bereicherung der Behandlung von Mensch und Tier sowie den Schutz von Pflanzen Mittel bereitzustellen, die nicht nur eine starke Wirksamkeit besitzen, sondern auch im Gegensatz zu anderen Arzneimitteln bzw. Pflanzenschutzmitteln verringerte Nebenwirkungen zeigen und damit eine geringere Gesundheitsgefahr für den Menschen bedeuten.

Aufgabe der vorliegenden Erfindung ist es daher, eine Substanz bereitzustellen, die bei Infektionen durch Viren, Pilze und Parasiten bei Menschen und Tieren und die oben angegebenen Bedingungen erfüllt.

Diese Aufgabe wird in völlig überraschender Weise durch die in Anspruch 1 definierte Stoffgruppe gelöst. Diese Stoffgruppe zeigt sowohl eine antiinfektiöse Wirkung gegen Viren, Pilze und ein- und mehrzellige Parasiten. Im Sinne dieser Erfindung ist die streng wissenschaftliche Definition von Parasiten anzuwenden. Das bedeutet, daß unter einzelligen Parasiten ausschließlich Protozoen verstanden werden.

Die erfindungsgemäß verwendeten phosphororganischen Verbindungen entsprechen der allgemeinen Formel (I): 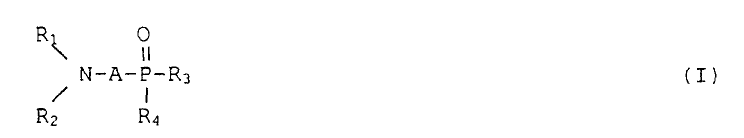 in der R₁ und R₂ gleich oder verschieden sind und aus der Gruppe ausgewählt sind, die aus Wasserstoff, substituiertem und unsubstituiertem Alkyl, substituiertem und unsubstituiertem Hydroxyalkyl, substituiertem und unsubstituiertem Alkenyl, substituiertem und unsubstituiertem Alkinyl, substituiertem und unsubstituiertem Aryl, substituiertem und unsubstituiertem Acyl, substituiertem und unsubstituiertem Cycloalkyl, substituiertem und unsubstituiertem Aralkyl, substituiertem und unsubstituiertem heterocyclischen Rest, Halogen, OX₁ und OX₂ besteht,
wobei X₁ und X₂ gleich oder verschieden sein können und aus der Gruppe ausgewählt sind, die aus Wasserstoff, substituiertem und unsubstituiertem Alkyl, substituiertem und unsubstituiertem Hydroxyalkyl, substituiertem und unsubstituiertem Alkenyl, substituiertem und unsubstituiertem Alkinyl, substituiertem und unsubstituiertem Aryl, substituiertem und unsubstituiertem Acyl, substituiertem und unsubstituiertem Cycloalkyl, substituiertem und unsubstituiertem Aralkyl, substituiertem und unsubstituiertem heterocyclischen Rest besteht,
A aus der Gruppe ausgewählt ist, die aus einem Alkylenrest, einem Alkenylenrest und einem Hydroxyalkylenrest besteht,
R₃ und R₄ unabhängig aus der Gruppe ausgewählt sind, die aus Wasserstoff, substituiertem und unsubstituiertem C₁₋₂₆-Alkyl, substituiertem und unsubstituiertem Hydroxy-C₁₋₂₆-alkyl, substituiertem und unsubstituiertem Aryl, substituiertem und unsubstituiertem Acyl, substituiertem und unsubstituiertem Aralkyl, substituiertem und unsubstituiertem C₁₋₂₆-Alkenyl, substituiertem und unsubstituiertem C₁₋₂₆-Alkinyl, substituiertem und unsubstituiertem Cycloalkyl, substituiertem und unsubstituiertem heterocyclischem Rest, Halogen, OX₃ und OX₄ besteht,
wobei X₃ und X₄ unabhängig aus der Gruppe ausgewählt sind, die aus Wasserstoff, substituiertem und unsubstituiertem C₁₋₂₆-Alkyl, substituiertem und unsubstituiertem Hydroxyl-C₁₋₂₆-alkyl, substituiertem und unsubstituiertem Aryl, substituiertem und unsubstituiertem Aralkyl, substituiertem und unsubstituiertem C₁₋₂₆-Alkenyl, substituiertem und unsubstituiertem C₁₋₂₆-Alkinyl, substituiertem und unsubstituiertem Cycloalkyl, substituiertem und unsubstituiertem heterocyclischem Rest, einem Silyl, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, besteht,
und deren pharmazeutisch akzeptablen Salze, Ester und Amide und Salze der Ester.

Bevorzugt sind insbesondere die Phosphonsaurederivate.

Insbesondere eignen sich die Verbindungen, die die folgende Formel (II) haben: 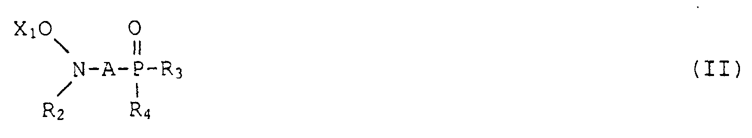 entsprechen, wobei
X₁ aus der Gruppe ausgewählt ist, die aus Wasserstoff, substituiertem oder unsubstituiertem Acyl, substituiertem oder unsubstituiertem Alkyl, substituiertem oder unsubstituiertem Aryl, substituiertem oder unsubstituiertem Aralkyl, substituiertem oder unsubstituiertem Cycloalkyl, substituiertem oder unsubstituiertem heterocyclischen Rest besteht;
R₂, R₃, R₄ und A die gleiche Bedeutung wie in Formel (I) haben.

Besonders bevorzugt ist A eine Kette aus drei Kohlenstoffatomen, die das Stickstoffatom mit dem Phosphoratom verbindet.

Insbesondere sind Verbindungen der Formel (II) bevorzugt, für die R₂ = Acyl, insbesondere ein Acetyl, R₃ = Wasserstoff, Methyl oder Ethyl, R₄ = Wasserstoff, Methyl, Ethyl oder OX₄ mit X₄ = Wasserstoff, Natrium, Kalium, Methyl, Ethyl, X₁ = H und A = Alkylen, Alkenylen oder Hydroxyalkylen ist. Besonders gute Ergebnisse werden mit R₂ = Formyl oder Acetyl und A = Propylen, Propenylen oder Hydroxypropylen erzielt.

Insbesondere sind ferner Verbindungen bevorzugt, in denen R₃ eine Alkyl-, Hydroxyalkyl-, Alkinyl- oder Alkenylgruppe mit 16 oder 18 Kohlenstoffatomen oder OX₃ ist, wobei X₃ eine Alkyl-, Alkinyl-, Hydroxyalkyl- oder Alkenylgruppe mit 16 oder 18 Kohlenstoffatomen ist, R₄ eine Alkyl-, Alkinyl-, Hydroxyalkyl- oder Alkenylgruppe mit 16 oder 18 Kohlenstoffatomen oder OX₄ ist, wobei X₄ eine Alkyl-, Alkinyl-, Hydroxyalkyl- oder Alkenylgruppe mit 16 oder 18 Kohlenstoffatomen ist.

Besonderheiten der obigen Definitionen und geeignete Beispiele dafür werden nachfolgend angegeben:
"Acyl" ist ein Substituent, der von einer Säure stammt, wie von einer organischen Carbonsäure, Kohlensäure, Carbaminsäure oder der den einzelnen vorstehenden Säuren entsprechenden Thiosäure oder Imidsäure, oder von einer organischen Sulfonsäure, wobei diese Säuren jeweils aliphatische, aromatische und/oder heterocyclische Gruppen im Molekül umfassen sowie Carbamoyl oder Carbamimidoyl.

Geeignete Beispiele für diese Acylgruppen werden nachfolgend angegeben.

Als aliphatische Acylgruppen werden von einer aliphatischen Säure stammende Acylreste bezeichnet, zu denen die folgenden gehören:
Alkanoyl (z.B. Formyl, Acetyl, Propionyl, Butyryl, Isobutyryl, Valeryl, Isovaleryl, Pivaloyl etc.);
Alkenoyl (z. B. Acryloyl, Methacryloyl, Crotonoyl etc.); Alkylthioalkanoyl (z.B. Methylthioacetyl, Ethylthioacetyl etc.)
Alkansulfonyl (z.B. Mesyl, Ethansulfonyl, Propansulfonyl etc.);
Alkoxycarbonyl (z.B. Methoxycarbonyl, Ethoxycarbonyl, Propoxycarbonyl, Isopropoxycarbonyl, Butoxycarbonyl, Isobutoxycarbonyl etc.);
Alkylcarbamoyl (z.B. Methylcarbamoyl etc.);
(N-Alkyl)-thiocarbamoyl (z.B. (N-Methyl)-thiocarbamoyl etc.);
Alkylcarbamimidoyl (z.B. Methylcarbamimidoyl etc.);
Oxalo;
Alkoxalyl (z.B. Methoxalyl, Ethoxalyl, Propoxalyl etc.).

Bei den obigen Beispielen für aliphatische Acylgruppen kann der aliphatische Kohlenwasserstoffteil, insbesondere die Alkylgruppe bzw. der Alkanrest, ggf. einen oder mehrere geeignete Substituenten aufweisen, wie Amino, Halogen (z.B. Fluor, Chlor, Brom etc.), Hydroxy, Hydroxyimino, Carboxy, Alkoxy (z.B. Methoxy, Ethoxy, Propoxy etc.), Alkoxycarbonyl, Acylamino (z.B. Benzyloxycarbonylamino etc.), Acyloxy (z.B. Acetoxy, Benzoyloxy etc.) und dergleichen; als bevorzugte aliphatische Acylreste mit solchen Substituenten sind z.B. mit Amino, Carboxy, Amino und Carboxy, Halogen, Acylamino oder dergleichen substituierte Alkanoyle zu nennen.

Als aromatische Acylreste werden solche Acylreste bezeichnet, die von einer Säure mit substituierter oder nicht substituierter Arylgruppe stammen, wobei die Arylgruppe Phenyl, Toluyl, Xylyl, Naphthyl und dergleichen umfassen kann; geeignete Beispiele werden nachfolgend angegeben:
Aroyl (z.B. Benzoyl, Toluoyl, Xyloyl, Naphthoyl, Phthaloyl etc.);
Aralkanoyl (z.B. Phenylacetyl etc.);
Aralkenoyl (z.B. Cinnamoyl etc.);
Aryloxyalkanoyl (z.B. Phenoxyacetyl etc.);
Arylthioalkanoyl (z.B. Phenylthioacetyl etc.);
Arylaminoalkanoyl (z.B. N-Phenylglycyl, etc.);
Arensulfonyl (z.B.Benzolsulfonyl, Tosyl bzw. Toluolsulfonyl, Naphthalinsulfonyl etc.);
Aryloxycarbonyl (z.B. Phenoxycarbonyl, Naphthyl-oxycarbonyl etc.);
Aralkoxycarbonyl (z.B. Benzyloxycarbonyl etc.);
Arylcarbamoyl (z.B. Phenylcarbamoyl, Naphthylcarbamoyl etc.);
Arylglyoxyloyl (z.B. Phenylglyoxyloyl etc.).

Bei den vorstehenden Beispielen für aromatische Acylreste kann der aromatische Kohlenwasserstoffteil (insbesondere der Arylrest) und/oder der aliphatische Kohlenwasserstoffteil (insbesondere der Alkanrest) ggf. ein oder mehrere geeignete Substituenten aufweisen, wie solche, die als geeignete Substituenten für die Alkylgruppe bzw. den Alkanrest bereits angegeben wurden. Insbesondere und als Beispiel für bevorzugte aromatische Acylreste mit besonderen Substituenten werden mit Halogen und Hydroxy oder mit Halogen und Acyloxy substituiertes Aroyl und mit Hydroxy, Hydroxyimino, Dihalogenalkanoyloxyimino substituiertes Aralkanoyl angegeben sowie
Arylthiocarbamoyl (z.B. Phenylthiocarbamoyl etc.);
Arylcarbamimidoyl (z.B. Phenylcarbamimidoyl etc.).

Als heterocyclischer Acylrest wird ein Acylrest verstanden, der von einer Säure mit heterocyclischer Gruppe stammt; dazu gehören:
Heterocyclisches Carbonyl, bei dem der heterocyclische Rest ein aromatischer oder aliphatischer 5-bis 6-gliedriger Heterocyclus mit zumindest einem Heteroatom aus der Gruppe Stickstoff, Sauerstoff und Schwefel ist (z.B. Thiophenyl, Furoyl, Pyrrolcarbonyl, Nicotinoyl etc.);
Heterocyclus-Alkanoyl, bei dem der heterocyclische Rest 5- bis 6-gliedrig ist und zumindest ein Heteroatom aus der Gruppe Stickstoff, Sauerstoff und Schwefel aufweist (z.B. Thiophenyl-acetyl, Furylacetyl, Imidazolylpropionyl, Tetrazolylacetyl, 2-(2-Amino-4-thiazolyl)-2-methoxyiminoacetyl etc.) und dergleichen.

Bei den obigen Beispielen für heterocyclische Acylreste kann der Heterocyclus und/oder der aliphatische Kohlenwasserstoffteil ggf. einen oder mehrere geeignete Substituenten aufweisen, wie die gleichen, die als geeignet für Alkyl- und Alkangruppen angegeben wurden.

"Alkyl" ist ein gerad- oder verzweigtkettiger Alkylrest mit bis zu 9 Kohlenstoffatomen, soweit nicht anders definiert, wie Methyl, Ethyl, Propyl, Isopropyl, Butyl, Isobutyl, tert.-Butyl, Pentyl, Hexyl und dergleichen.

"Hydroxylalkyl" ist ein gerad- oder verzweigtkettiger Alkylrest mit bis zu 9 Kohlenstoffen, soweit nicht anders definiert, der mindestens eine Hydroxylgruppe aufweist, bevorzugt ein oder zwei Hydroxylgruppen.

Zu "Alkenyl" gehören gerad- oder verzweigtkettige Alkenylgruppen mit bis zu 9 Kohlenstoffatomen, soweit nicht anders definiert, wie z.B. Vinyl, Propenyl (z.B. 1-Propenyl, 2-Propenyl), 1-Methylpropenyl, 2-Methylpropenyl, Butenyl, 2-Ethylpropenyl, Pentenyl, Hexenyl.

Zu "Alkinyl" gehören gerad- oder verzweigtkettige Alkinylgruppen mit bis zu 9 Kohlenstoffatomen, soweit nicht anders definiert.

Cycloalkyl steht vorzugsweise für ein ggfs. substituiertes C3-C7-Cycloalkyl; als mögliche Substituenten sind u.a. Alkyl, Alkenyl, Alkinyl, Alkoxy (z.B. Methoxy, Ethoxy etc.), Halogen (z.B. Fluor, Chlor, Brom etc.), Nitro und dergleichen geeignet.

Aryl ist ein aromatischer Kohlenwasserstoffrest, wie Phenyl Naphthyl usw., der ggf. einen oder mehrere geeignete Substituenten aufweisen kann, wie Alkyl, Alkenyl, Alkinyl, Alkoxy (z.B. Methoxy, Ethoxy etc.), Halogen (z.B. Fluor, Chlor, Brom etc.), Nitro und dergleichen.

Zu "Aralkyl" gehören Mono-, Di-, Triphenylalkyle wie Benzyl, Phenethyl, Benzhydryl, Trityl und dergleichen, wobei der aromatische Teil ggf. ein oder mehrere geeignete Substituenten aufweisen kann wie Alkoxy (z.B. Methoxy, Ethoxy etc.), Halogen (z.B. Fluor, Chlor, Brom etc.), Nitro und dergleichen.

Zu "Alkylen" gehören gerad- oder verzweigtkettige Alkylengruppen, die bis zu 9 Kohlenstoffatome aufweisen und durch die Formel

-(CₙH₂ₙ)-

wiedergegeben werden können, in der n eine ganze Zahl von 1 bis 9 ist, wie Methylen, Ethylen, Trimethylen, Methylethylen, Tetramethylen, 1-Methyltrimethylen, 2-Ethylethylen, Pentamethylen, 2-Methyltetramethylen, Isopropylethylen, Hexamethylen, und dergleichen; bevorzugte Alkylenreste haben bis zu 4 Kohlenstoffatome und besonders bevorzugt werden Reste mit 3 Kohlenstoffatomen wie z.B. Trimethylen. Die Wasserstoffatome können durch andere Substituenten, wie zum Beispiel Halogenreste, ersetzt sein.

Zu "Alkenylen" gehören gerad- oder verzweigtkettige Alkenylengruppen mit bis zu 9 Kohlenstoffatomen, die durch die Formel

-(CₙH₂ₙ₋₂)-

wiedergegeben werden können, in der n eine ganze Zahl von 2 bis 9 ist, wie z.B. Vinylen, Propenylen (z.B. 1-Propenylen, 2-Propenylen), 1-Methylpropenylen, 2-Methylpropenylen, Butenylen, 2-Ethylpropenylen, Pentenylen, Hexenylen und dergleichen; besonders bevorzugt kann der Alkenylenrest bis zu 5 Kohlenstoffatome aufweisen und insbesondere 3 Kohlenstoffatome wie z.B. 1-Propenylen. Die Wasserstoffatome können durch andere Substituenten, wie zum Beispiel Halogenreste, ersetzt sein.

Zu "Hydroxyalkylen" können gerad- oder verzweigtkettige Alkylenreste gehören, die bis zu 9 Kohlenstoffatome aufweisen, wobei mindestens ein ausgewähltes Kohlenstoffatom mit einer Hydroxygruppe substituiert ist; diese Reste können durch die Formel

-(CₙH_{2n-z})(OH)_{z}-

wiedergegeben werden, in der n eine ganze Zahl von 1 bis 9 ist und z eine ganze Zahl ist, für die 1 ≤ z ≤ n gilt. Zu geeigneten Beispielen für solche Hydroxyalkylengruppen gehören Hydroxymethylen, Hydroxyethylen (z.B. 1-Hydroxyethylen und 2-Hydroxyethylen), Hydroxytrimethylen (z.B. 1-Hydroxytrimethylen, 2-Hydroxytrimethylen und 3-Hydroxytrimethylen), Hydroxytetramethylen (z.B. 2-Hydroxytetramethylen), 2-Hydroxy-2-methyltrimethylen, Hydroxypentamethylen (z.B. 2-Hydroxypentamethylen), Hydroxyhexamethylen (z.B. 2-Hydroxyhexamethylen) und dergleichen. Besonders bevorzugt wird ein niederes Hydroxyalkylen mit bis zu 4 Kohlenstoffatomen und insbesondere ein solches mit 3 Kohlenstoffatomen wie z.B. 2-Hydroxytrimethylen. Die Wasserstoffatome können durch andere Substituenten, wie zum Beispiel Halogenreste, ersetzt sein.

Vorzugsweise können die Reste X₃ und X₄ so gewählt werden, daß Ester an der Phosphinogruppe oder Phosphonogruppe gebildet werden. Zu geeigneten Beispielen für solche Ester gemäß der Formeln (I) und (II) zählen Alkylester (z.B. Methylester, Ethylester, Propylester, Isopropylester, Butylester, Isobutylester, Hexylester, Hexadecanylester, Octadecanylester etc.);
Aralkylester (Benzylester, Phenethylester, Benzhydrylester, Tritylester etc.);
Arylester (z.B. Phenylester, Tolylester, Naphthylester etc.); Aroylalkylester (z.B. Phenacylester etc.); und Silylester (z.B. von Trialkylhalogensilyl, Dialkyldihalogensilyl, Alkyltrihalogensilyl, Dialkylarylhalogensilyl, Trialkoxyhalogensilyl, Dialkylaralkylhalogensilyl, Dialkoxydihalogensilyl, Trialkoxyhalogensilyl etc.) und dergleichen.

Beim obigen Ester kann der Alkan- und/oder Arenteil wahlweise zumindest einen geeigneten Substituenten aufweisen wie Halogen, Alkoxy, Hydroxy, Nitro oder dergleichen.

Bevorzugt sind X₃ und X₄ ein Metall der ersten, zweiten oder dritten Hauptgruppe des Periodensystems, Ammonium, substituiertem Ammonium, oder Ammoniumverbindungen, die sich von Ethylendiamin oder Aminosäuren ableiten. D.h. es werden die Salzverbindungen der phosphororganischen Verbindungen mit organischen oder anorganischen Basen (z.B. Natriumsalz, Kaliumsalz, Calciumsalz, Aluminiumsalz, Ammoniumsalz, Magnesiumsalz, Triethylaminsalz, Ethanolaminsalz, Dicyclohexylaminsalz, Ethylendiaminsalz, N,N'-Dibenzylethylendiaminsalz etc.) sowie Salze mit Aminosäuren (z.B. Argininsalz, Asparaginsäuresalz, Glutaminsäuresalz etc.) und dergleichen gebildet.

Die erfindungsgemäß verwendeten Verbindungen gemäß der Formeln (I) oder (II) können in ihrer protonierten Form als Ammoniumsalz organischer oder anorganischer Säuren, wie Salzsäure, Bromwasserstoffsäure, Schwefelsäure, Salpetersäure, Methansulfonsäure, p-Toluolsulfonsäure, Essigsäure, Milchsäure, Maleinsäure, Fumarsäure, Oxalsäure, Weinsäure, Benzoesäure, etc. vorliegen.

Die erfindungsgemäß verwendeten Verbindungen der Formeln (I) oder (II) lassen beispielsweise für Doppelbindungen enthaltende oder chirale Gruppen R₁, R₂, R₃, R₄, X₁, X₂, X₃, X₄ oder A das Auftreten räumlicher Isomere zu. Die erfindungsgemäße Verwendung der Verbindungen umfaßt alle räumlichen Isomere sowohl als Reinstoffe als auch in Form ihrer Mischungen.

Die phosphororganischen Verbindungen sind insbesondere für die therapeutische und prophylaktischen Behandlung von Infektionen bei Mensch und Tier geeignet, die durch Viren, ein- und mehrzellige Parasiten und Pilze hervorgerufen werden.

Die Verbindungen sind gegen einzellige Parasiten (Protozoen) wirksam, insbesondere gegen Erreger der Malaria und der Schlafkrankheit sowie der Chagas-Krankheit, der Toxoplasmose, der Amöbenruhr, der Leishmaniosen, der Trichomoniasis, der Pneumozystose, der Balantidiose, der Kryptosporidiose, der Sarkozystose, der Akanthamöbose, der Naeglerose, der Kokzidiose, der Giardiose und der Lambliose.

Sie sind daher insbesondere als Malariaprophylaxe und als Prophylaxe der Schlafkrankheit sowie der Chagas-Krankheit, der Toxoplasmose, der Amöbenruhr, der Leishmaniosen, der Trichomoniasis, der Pneumozystose, der Balantidiose, der Kryptosporidiose, der Sarkozystose, der Akanthamöbose, der Naeglerose, der Kokzidiose, der Giardiose und der Lambliose geeignet.

Es können auch Kombination mit einem Antibiotikum zur Behandlung der obengenannten Erkrankungen eingesetzt werden. Für Kombinationspräparate mit anderen Antiinfektiva eignen sich insbesondere Isoniazid, Rifampicin, Ethambutol, Pyrazinamid, Streptomycin, Protionamid und Dapson zur Behandlung der Tuberkulose.

Die erfindungsgemäßen Wirkstoffe sind ferner insbesondere bei Infektionen mit folgenden Viren einsetzbar:
Parvoviridae: Parvoviren, Dependoviren, Densoviren, Adenoviridae: Adenoviren, Mastadenoviren, Aviadenoviren, Papovaviridae: Papovaviren, insbesondere Papillomaviren (sogenannte Warzenviren), Polyomaviren, insbesondere JC-Virus, BK-Virus, und Miopapovaviren,
Herpesviridae: Alle Herpesviren, insbesondere Herpes-Simplex-Viren, der Varizellen/Zoster-Viren, menschlicher Zytomegalievirus, Epstein-Barr-Viren, alle humanen Herpesviren, humanes Herpesvirus 6, Humanes Herpesvirus 7, humanes Herpesvirus 8, Poxviridae:Pockenviren, Orthopox-, Parapox-, Molluscum-Contagiosum-Virus, Aviviren, Capriviren, Leporipoxviren, alle primär hepatotropen Viren, Hepatitisviren: Hepatitis-A-Viren, Hepatitis-B-Viren, Hepatitis-C-Viren, Hepatitis-D-Viren, Hepatitis-E-Viren, Hepatitis-F-Viren, Hepatits-G-Viren, Hepadnaviren: sämtliche Hepatitisviren, Hepatitis-B-Virus, Hepatitis-D-Viren,
Picornaviridae: Picornaviren, alle Enteroviren, alle Polioviren, alle Coxsackieviren, alle Echoviren, alle Rhinoviren, Hepatitis-A-Virus, Aphthoviren,
Calciviridae: Hepatitis-E-Viren,
Reoviridae: Reoviren, Orbiviren, Rotaviren,
Togaviridae: Togaviren, Alphaviren, Rubiviren, Pestiviren, Rubellavirus,
Flaviviridae: Flaviviren, FSME-Virus, Hepatitis-C-Virus, Orthomyxoviridae: Alle Influenzaviren,
Paramyxoviridae: Paramyxoviren, Morbillivirus, Pneumovirus, Masernvirus, Mumpsvirus,
Rhabdoviridae: Rhabdoviren, Rabiesvirus, Lyssavirus, viskuläres Stomatitisvirus,
Coronaviridae: Coronaviren,
Bunyaviridae: Bunyaviren, Nairovirus, Phlebovirus, Uukuvirus, Hantavirus, Hantaanvirus,
Arenaviridae: Arenaviren, lymphozytäres Choriomeningitis-Virus,
Retroviridae: Retroviren, alle HTL-Viren, humanes T-cell Leukämievirus, Oncornaviren, Spumaviren, Lentiviren, alle HI-Viren,
Filoviridae: Marburg- und Ebolavirus,
Slow-virus-Infektionen,
Onkoviren und Leukämieviren.

Die erfindungsgemäß verwendeten phosphororganischen Verbindungen sind somit zur Bekämpfung folgender viraler Infekte geeignet:

Eradikation von Papillomaviren zur Vorbeugung von Tumoren, insbesondere von Tumoren der Geschlechtsorgane verursacht durch Papillomaviren beim Menschen, Eradikation von JC-Viren und BK-Viren, Eradikation von Herpesviren, Eradikation humaner Herpesviren 8 zur Behandlung der Kaposi-Sarkoma, Eradikation von Zytomegalie-Viren vor Transplantationen, Eradikation von Eppstein-Barr-Viren vor Transplantation und zur Vorbeugung von Eppstein-Barr-Viren-assozierten Tumoren, Eradikation von Hepatitisviren zur Behandlung von chronischen Leber-Erkrankungen und zur Vorbeugung von Lebertumoren und Leberzirrhosen, Eradikation von Coxsackieviren bei Kardiomyopathien, Eradikation von Coxsackieviren bei Diabetes-mellitus-Patienten, Eradikation von Immunschwäche-Viren in Mensch und Tier, Behandlung von Begleitinfektionen in AIDS-Patienten, Behandlung von Entzündungen viraler Genese des Respirationstraktes (Larynxpapillome, Hyperplasien, Rhinitis, Pharyngitis, Bronchitis, Pneumonien), der Sinnesorgane (Keratokonjunktivitis), des Nervensystems (Poliomyelitis, Meningoenzephalitis, Enzephalitis, subakute sklerosierende Panenzephalitis SSPE, progressive multifokale Leukoenzephalopathie, Lymphozytäre Choriomeningitis), des Magen-Darm-Traktes (Stomatitis, Gingivostomatitis, Ösophagitis, Gastritis, Gastroenteritis, Durchfallerkrankungen), der Leber und des Gallensystems (Hepatitis, Cholangitis, hepatozelluläres Karzinom), des lymphatischen Gewebes (Mononukleose, Lymphadenitis), des hämatopoetischen Systems, der Geschlechtsorgane (Mumpsorchitis), der Haut (Warzen, Dermatitis, Herpes labialis, Fieberbläschen, Herpes Zoster, Gürtelrose), der Schleimhäute (Papillome, Konjunktivapapillome, Hyperplasien, Dysplasien), des Herz-Blutgefäß-Systems (Arteriitis, Myokarditis, Endokarditis, Perikarditis), des Nieren-Harnweg-Systems, der Geschlechtsorgane (Anogenitale Läsionen, Warzen, Genitalwarzen, spitzen Kondylome, Dysplasien, Papillome, Zervixdysplasien, Condylomata acuminata, Epidermodysplasia verruciformis), der Bewegungsorgane (Myositis, Myalgien), Behandlung der Maul- und Klauenseuche der Paarhufer, des Colorado-Zeckenfiebers, des Dengue-Syndroms, des hämorrhagisches Fiebers, der Frühsommermeningoenzephalitis (FSME) und des Gelbfiebers.

Die beschriebenen Verbindungen, d.h. die phosphororganischen Verbindungen nach Formel (I) und (II) und Ester und Amide derselben an der Phosphinogruppe oder Phosphonogruppe sowie Salze derselben zeigen eine starke zytotoxische Wirksamkeit gegenüber ein- und mehrzelligen Parasiten, insbesondere gegenüber den Erregern der Malaria und der Schlafkrankheit. Demgemäß sind die erfindungsgemäß verwendeten Verbindungen für die Behandlung von Infektionskrankheiten brauchbar, die durch Viren, Parasiten und Pilze bei Mensch und Tier verursacht werden. Die Verbindungen sind auch für den Einsatz zur Vorbeugung von Erkrankungen, die durch Viren, Parasiten und Pilze hervorgerufen werden, insbesondere als Malariaprophylaxe und als Schlafkrankheitsprophylaxe geeignet.

Die erfindungsgemäß verwendeten phosphororganischen Verbindungen, hierzu gehoren im allgemeinen pharmazeutisch verträgliche Salze, Amide, Ester, ein Salz eines solchen Esters, oder aber Verbindungen, die bei Applikation die erfindungsgemäß verwendeten Verbindungen als Stoffwechselprodukte oder Abbauprodukte bereitstellen, auch "Prodrugs" genannt, können für die Verabreichung in irgendeiner geeigneten Weise analog zu bekannten antiinfektiös wirkenden Mitteln (gemischt mit einem nicht toxischen pharmazeutisch akzeptablen Träger) zubereitet werden.

Zu pharmazeutisch akzeptablen Salzen der Verbindungen gehören Salze, die die erfindungsgemäß verwendeten Verbindungen der Formeln (I) und (II) in ihrer protonierten Form als Ammoniumsalz anorganischer oder organischer Säuren, wie Salzsäure, Schwefelsäure, Zitronensäure, Maleinsäure, Fumarsäure, Weinsäure, p-Toluolsulfonsäure, bilden.

Pharmazeutisch besonders geeignet sind auch die Salze, die durch geeignete Auswahl von X₃ und X₄ gebildet werden, wie Natriumsalz, Kaliumsalz, Calciumsalz, Ammoniumsalz, Ethanolaminsalz, Triethylaminsalz, Dicyclohexylaminsalz und Salze einer Aminosäure wie Argininsalz, Asparaginsäuresalz, Glutaminsäuresalz.

Die Aktivität der Substanzen wird in einem Versuchssystem bestimmt. Dieses System beruht auf die Messung der Inhibition des Wachstums von Parasiten, Viren, Pilzen oder Pflanzen in vitro. Hierzu werden zum Teil Versuchsverfahren verwendet, die dem Fachmann bekannt sind.

Zum Beispiel wird zur Bestimmung der Antimalaria Aktivität die Inhibition des Wachstums von Malaria Parasiten in Blutkulturen bestimmt.

Die Bestimmung der antiviralen Aktivität beruht auf Inhibition der Bildung von viralen Elementen in Zellkulturen.

Die Bestimmung der fungiziden Aktivität beruht auf Inhibition des Wachstums von Pilzen auf Nährböden und in Flüssigkulturen.

Einige der Mikroorganismen, die untersucht werden sollen können nur in Tiermodellen untersucht werden. Hier werden wir dann die entsprechenden Modelle anwenden.

Substanzen, die eine Wirksamkeit in den in vitro Messsystemen zeigen, weiter in in vivo Modellen weiter untersucht.
Die antiparasitäre, antivirale oder fungizide Aktivität wird in den entsprechenden Tiermodelle weiter evaluiert.

Die pharmazeutisch wirksamen Mittel können in Form von pharmazeutischen Zubereitungen in Dosierungseinheiten zubereitet werden. Dies bedeutet, daß die Zubereitung in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. C14-Alkohol mit C16-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin, enthalten.

Die Wirkstoffe der Formeln (I) und (II) sollen in den oben aufgeführten pharmazeutischen Zubereitungen, vorzugsweise in einer Konzentration von etwa 0,1 bis 99,5 Gew.-%, vorzugsweise von etwa 0,5 bis 95 Gew.-%, der Gesamtmischung vorhanden sein.

Die pharmazeutischen Zubereitungen können außer den Verbindungen der Formeln (I) und (II) auch weitere pharmazeutische Wirkstoffe enthalten.

Die Verbindungen können mit bisher beschriebenen Substanzen mit antibakterieller, antiviraler, antimyktoischer und antiparasitärer Eigenschaften verwendet werden. Hierzu gehören insbesondere Verbindungen, die bereits in der Therapie Anwendung gefunden haben oder noch angewendet werden. Hierzu sind insbesondere geeignet Stoffe, die in der in der Roten Liste oder in Simon/Stille, Antibiotika-Therapie in Klinik und Praxis, 9.Auflage 1998 Schattauer Verlag, oder unter
http:/www.customs.treas.gov/imp-exp/rulings/harmoniz/hrm129.html im Internet mitaufgeführt sind. Insbesondere können die Derivate mit Penicillin, Benzylpenicillin (Penicillin G), Phenoxypenicillin, Isoxazolylpenicillin, Aminopenicillin, Ampicillin, Amoxixillin, Bacampicillin, Carboxypenicillin, Ticarcillin, Temocillin, Acyalaminopenicillin, Azlocillin, Mezlocillin, Piperacillin, Apalcillin, Mecillinam, Cephalosporin, der Cefazolin-Gruppe, der Cefuroxim-Grup-pe, der Cefoxitin-Gruppe, Cefoxitin, Cefotetan, Cefmetazol, Latamoxef, Flomoxef, der Cefotaxim-Guppe, Cefozidim, der Ceftazidim-Gruppe, Ceftazidim, Cefpirom, Cefepim, den übrigen Cephalosporinen, Cefsulodin, Cefoperazon, Oralcephalosporinen der Cefalexin-Gruppe, Loracarbef, Cefprozil, neuen Oralcephalosporinen mit erweitertem Spektrum, Cefixim, Cefpodoxim-Proxetil, Cefuroxim-Axetil, Cefetamet, Cefotiam-Hexetil, Cefdinir, Ceftibuten, anderen β-Lactam-Antibiotika, Carbapenem, Imipenem /Cilastatin, Meropenem, Biapenem, Aztreonam, β-Lacta-mase-Hemmer, Clavulansäure/Amoxicillin, Clavulansäure/Ticar-cillin, Sulbactam/Ampicillin, Tazobactam/Piperacillin, Tetracycline, Oxytetracyclin, Rolitetraxyxlin, Doxycyclin, Minocyclin, Chloramphenicol, Aminoglykosiden, Gentamicin, Tobramycin, Netilmicin, Amikacin, Spectinomyxin, Makrolid, Erythromycin, Clarithromycin, Roxithromycin, Azithromycin, Dirithromycin, Spiramycin, Josamycin, Lincosamid, Clindamycin, Fusidinsäure, Glykopeptid-Antibiotika, Vancomycin, Teicoplanin, Pristinamycin-Derivaten, Fosfomycin, antimikrobiellen Folsäureantagonisten, Sulfonamiden, Co-Trimoxazol, Trimethoprim, anderen Diaminopyrimidin-Sulfonamid-Kombinationen, Nitrofuranen, Nitrofurantoin, Nitrofurazon, Gyrase-Hemmern (Chinolone), Norfloxacin, Ciprofloxacin, Ofloxacin, Sparfloxacin, Enoxacin, Fleroxacin, Pefloxacin, Lomefloxacin, 1-Cyclopropyl-7-(2,8-diazabicyclo-[4.3.0)]non-8-yl)-6-fluoro-8-chloro-1,4-dihydro-4-oxo-3-chinolincarbonsäurehydrochlorid(Bay Y3118), Nitroimidazolen, antimykobakteriellen Mitteln, Isoniazid, Rifampicin, Rifabutin, Ethambutol, Pyrazinamid, Streptomycin, Capreomycin, Prothionamid, Terizidon, Dapson, Clofazimin, Lokalantibiotika, Bacitracin, Tyrothricin, Polymyxine, Neomycin, Kanamycin, Paromomycin, Mupirocin, antiviralen Mitteln, Acyclovir, Ganciclovir, Azidothymidin, Didanosin, Zalcitabin, Thiacytidin, Stavudin, Ribavirin, Idoxuridin, Trifluridin, Foscarnet, Amantadin, Interferone, Tibol-Derivaten, Proteinase-Inhibitoren, Antimykotika, Polyenen, Amphothericin B, Nystatin, Natamycin, Azolen, Azolen zur septischen Therapie, Miconazol, Ketoconazol, Itraconazol, Fluconazol, Voriconazol (UK-109.496), Azolen für lokale Anwendung, Clotrimazol, Econazol, Isoconazol, Oxiconazol, Bifonazol, Flucytosin, Griseofulvin, Ciclopiroxolamin, Tolnaftat, Naftifin, Terbinafin, Amorolfin, Antrachinon, Betulinsäure, Semianthrachinon, Xanthon, Naphtochinon, Aryaminoalkohol, Chinin, Chinidin, Mefloquin, Halofantrin, Chloroquin, Amodiaquin, Acridin, Benzonaphthyridin, Mepacrin, Pyronaridin, Dapson, Sulfonamiden, Sulfadoxin, Sulfalen, Trimethoprim, Proguanil, Chlorproguanil, Diaminopyrimidine, Pyrimethamin, Primaquin, Aminochinoline, Tafenoquin(WR 238,605), Artemisinin, Dihydroartemisinin, 10b Artemether, Arteether, Atrtesunat, Atovaquon, Suramin, Melarsoprol, Nifurtmox, Stibogluconat-Natrium, Pentamidin, Amphotericin B, Metronidazol, Cliochinol, Mebendazol, Niclosamid, Praziquantel, Pyrantel, Tiabendazol, Diethylcarbamazin, Ivermectin, Bithionol, Oxamniquin, Metrifonat, Piperazin, Embonat.

Ferner können die phosphororganischen Verbindungen in den pharmazeutischen Mitteln in Kombination mit Sulfonamid, Sulfadoxin, Artemisinin, Atovaquon, Chinin, Chloroquin, Hydroxychloroquin, Mefloquin, Halofantrin, Pyrimethamin, Armesin, Tetracycline, Doxycyclin, Proguanil, Metronidazol, Praziquantil, Niclosamid, Mebendazol, Pyrantel, Tiabendazol, Diethylcarbazin, Piperazin, Pyrivinum, Metrifonat, Oxamniquin, Bithionol oder Suramin oder mehreren dieser Substanzen vorliegen.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionslösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Prämixe, Konzentrate, Granulate, Pellets, Tabletten, Boli, Kapseln, Aerosole, Sprays, Inhalate bei Mensch und Tier angewendet werden. Ferner können die erfindungsgemäß verwendeten Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die Wirkstoffe der Formeln (I) und (II) in Gesamtmengen von etwa 0,05 bis etwa 600, vorzugsweise 0,5 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 200, insbesondere 1 bis 60 mg/kg Körpergewicht. Es kann jedoch erforderlich sein, von den genannten Dosierungen abzuweichen, und zwar in Abhängigkeit von der Art und dem Körpergewicht des zu behandelnden Patienten, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch den Fachmann aufgrund seines Fachwissens erfolgen.

Die erfindungsgemäßen Verbindungen können in den üblichen Konzentrationen und Zubereitungen bei Tieren zusammen mit dem Futter bzw. mit Futterzubereitungen oder mit dem Trinkwasser gegeben werden.

### Beispiel 1

### Test der Wirksamkeit der Substanzen gegen Malaria in vivo

Die verschiedenen Derivate wurden nach dem modifizierten Peters' Test getestet. Die Substanzen wurden dabei in einem Viertel der halblethalen Dosis (LD50) appliziert. Bei dem Versuchsansatz wurden zehn Mäuse mit Plasmodium vinckeii, dem Erreger der Mäusemalaria, infiziert. Nach Bestätigung der Infektion durch Blutuntersuchung erfolgte die Behandlung in vier Mäusen. Als Kontrolle dienten sechs Mäuse, die nicht behandelt wurden. Die Behandlung mit 1000 mg/kg/d , 3-(N-Formyl-N-Hydroxylamino)-propylphosphonsäuremononatriumsalz über 3 Tage führte zu einer Abtötung der Parasiten im Blut der Mäuse. Die behandelte Gruppe war bereits nach einem Tag frei von lebenden Parasiten. Die Kontrollmäuse mußten am Tag 5 nach Infektion bei einer Parasitämie von > 80% getötet werden. Die behandelten Mäuse waren auch 8 Wochen nach Behandlungsende immer noch frei von Parasiten. Weitere Experimente zeigten eine Wirksamkeit von 50 mg/kg/d 3-(N-Formyl-N-hydroxylamino)-propylphosphonsäure-mononatriumsalz in Mäusen mit einer Parasitämie von 80%. Auch diese Mäuse waren nach 1 Tag frei von lebenden Parasiten.

### Beispiel 2

### Schutzwirkung vor Malaria beim Versuch mit infizierten Mäusen

Die Wirksamkeit der Verbindungen in vivo gegenüber Malaria wurde unter Heranziehen von 20 bis 25 g schweren männlichen Mäusen (BALB/c-Stamm) getestet. Einen Tag vor der Infektion wurden vier Mäuse intraperitoneal mit 50 mg/kg 3-(N-Formyl-N-hydroxylamino)-propylphosphonsäure-mononatriumsalz behandelt. Die Mäuse wurden dann mit Plasmodium vinckeii infiziert. Mäuse, die nicht mit der Substanz vorbehandelt wurden, dienten als Kontrolle. Es konnte in den behandelten Mäusen keine Infektion nachgewiesen wurden, während die Kontrollmäuse nach 5 Tagen mit einer Parasitämie über 80% getötet werden. Die behandelten Mäuse waren auch 8 Wochen nach der Infektion frei von Parasiten.

### Beispiel 3

### In vitro Zytotoxizität gegenüber Malaria Parasiten Zum Prinzip der IC50-Bestimmung nach Vial et al. (die Konzentration, bei der die Vitalität der Parasiten um die Hälfte reduziert wird)

Zur Bestimmung der IC50-Werte nach Vial et al. werden die Malaria-Parasiten zunächst für einen vollständigen 48-Stunden-Zyklus in Gegenwart von Inhibitoren kultiviert, in den anschließenden 24 Stunden wurde die Überlebensrate durch [³H]-Hypoxanthin-Einbau gemessen.

### Durchführung des Tests

Auf einer Mikrotiterplatte wird eine Verdünnungsreihe von 3-(N-Formyl-N-hydroxylamino)-propylphosphonsäuremononatriumsalz in 10-fach konzentrierten 20-µl-Aliquots vorgelegt. Dann werden zu jedem Well 180 µl Parasitensuspension in Kulturmedium zugefügt. Es werden asynchrone Kulturen mit ca. 0,4% Parasitämie und 2 % Hämatokrit verwendet. Anschließend werden die Mikrotiterplatten für 48 h inkubiert. Dann werden zu jedem Well 30 µl [³H]-Hypoxanthin zugefügt. Nach 24-stündiger Inkubation wurden die Zellen geerntet und die inkorporierte Radioaktivität wurde gemessen. In Figur 1 sind die Ergebnisse mit den Stämmen HB3 und Dd2 mit bekannten Resistenzen gegen andere Malaria-Medikamente dargestellt. In beiden Stämmen ergibt sich ein IC-50-Wert von ca. 100 µg/l. Die Resistenzen dieser Stämme sind:
Plasmodium falciparum HB3 (Honduras) ist gegen Pyrimethamin resistent.
Plasmodium falciparum Dd2 (Indochina) ist gegen Cloroquin, Chinin, Pyrimethamin, Cycloguanil und Sulfadoxin resistent.

Es wurden keine Kreuzresistenzen mit Anti-Malaria-Mitteln gefunden.

### Beispiel 4

### Herstellung von 3-Brompropylphosphonsäurediethylester

471 g (238 ml, 2,33 mol) 1,3-Dibrompropan und 77,6 g (81 ml, 0,467 mol) Triethylphosphit werden in 500 ml-Kolben vorgelegt und 30 min. auf 155-160°C erwärmt. Dabei werden 20 ml Ethylbromid (Sdp: 40°C) über einen Rückflußkühler und eine Destille unter Normaldruck abdestilliert. Das Einengen der Lösung im Vakuum (8 Torr (1,07·10³ Pa)) ergab 380 g (191 ml, 1,863 mol) 1,3 Dibrompropan (überschüssiges Edukt). aus dem zurückbleibenden gelben Öl konnten 88,1 g (0,34 mol) als farblose Flüssigkeit destilliert werden (Sdp: 96°C, 0,1 Torr (13,33 Pa)). Dies entspricht einer Ausbeute von 73 %. (Hewitt, Teese, Aust.J.Chem. 1984, 37, 205-10, US-Patent 4 206 156)
- ¹H-NMR(CDCl₃): δ= 4,08(quintett, J=7 Hz, 4H) , 1,33(t, J=7 Hz, 6H)
- ¹³C-NMR(CDCL₃): δ= 61,2(OCH₂CH₃), 33,10(J=18,3 Hz), 25,6(J=4,4 Hz), 24,14(J=120,6 Hz), 16,04(OCH₂CH₃)

### Beispiel 5

### Herstellung von 3-(N-hydroxyamino)-propylphosphonsäurediethylester

Zu einer Lösung von 55,6 g (0,8 mol) Hydroxylaminhydrochlorid in 100 ml Wasser werden unter Eiskühlung zunächst 32,0 g (0,8 mol) Natriumhydroxyd, gelöst in 75 ml Wasser, dann 75 ml Methanol und schließlich 25,5 g (0,098 mol) 3-Brompropylphosphonsäurediethylester zugetropft. Dies führt zu einer Trübung der Lösung. Nach 3-stündigem Rühren bei einer Temperatur von 40-45°C wird Methanol unter reduziertem Druck entfernt, die resultierende wäßrige Lösung mit NaHCO₃ gesättigt (pH=8), dreimal mit je 60 ml Toluol ausgeschüttelt (die Toluol-Phase wurde verworfen) und dann mit Chloroform ausgeschüttelt (1× mit 90 ml, 2× mit je 50 ml). Die leicht gelbliche Chloroform-Phase wird über MgSO₄ getrocknet. Nach dem Filtrieren des Trockenmittels wird die Lösung unter reduziertem Druck eingeengt. Es werden 15,43 g (0,0728 mol) 3-(N-hydroxyamino)-propylphosphonsäurediethylester als nahezu farbloses Öl erhalten. Dies entspricht einer Ausbeute von 74,3 %. (DE-A-27 33 658)
- ¹H-NMR(CDCl₃): δ= 5,94(breites s,2H), 4,13(quintett, J=7 Hz, 4H), 2,90 (t, J=7 Hz, 2H), 1,5-2,2(m,4H), 1,33(t,J=7 Hz,6H)
- ¹³C-NMR(CDCL₃): δ= 61,23(OCH₂CH₃), 53,34(NCH₂, J=15,9 Hz), 22,75(J=141,9 Hz), 19,77 Hz, 16,08 (OCH₂CH₃)

### Beispiel 6

### Herstellung von 3-(N-hydroxyamino)-propylphosphonsäure

12,9 g (0,0608 mol) 3-(N-hydroxyamino)-propylphosphonsäurediethylester und 130 ml konzentrierte HCl werden 6 h lang unter Rückfluß erhitzt (Ölbad-T.: 150°C). Die resultierende gelborange Lösung wird unter reduziertem Druck eingeengt. Das zurückbleibende Öl wird in 30 ml Wasser aufgenommen, mit 3 Löffeln Aktivkohle 30 min gerührt, von der Aktivkohle abfiltriert und die farblose Lösung im vollen Membranpumpenvakuum eingeengt. Nach Aufnehmen in 30 ml Wasser wird mit ca. 4,7 g (0,056 mol) NaHCO₃ ein pH-Wert von 4,0-4,5 eingestellt (ab pH=1,5 fällt Produkt aus). Abnutschen des weißen Feststoffs ergab 5,83 g 3-(Hydroxyamino)-propylphosphon-säure (Smp.: 160°C, Zersetzung). Dies entspricht einer Ausbeute von 61,8 %. (DE-A-27 33 658, Öhler Sythesis 1995, 539-543)
- ¹H-NMR (CDCl₃): δ= 3,49 (t, J=7,4 Hz, 2H), 2,1(m,2H), 1,82(m, PCH₂, 2H)
- ¹³C-NMR(CDCL₃): δ= 56,26 (NCH₂, J=15 Hz), 29,61 (PC, J=134 Hz), 22,37 (C-2, J=3,8 Hz)

### Beispiel 7

### Herstellung von 3-(N-formylhydroxyamino)-propylphosphonsäurediethylester

1,38 (0,030 mol) Ameisensäure werden bei Zimmertemperatur zu 2,04 g (0,020 mol) Essigsäureanhydrid zugetropft und bei gleicher Temperatur gerührt. Diese Lösung wird unter Eiskühlung zu 2,8 g (0,013 mol) 3-(N-hydroxyamino)-propylphosphonsäurediethylester, gelöst in Chloroform, zugegeben. Die Reaktionsmischung wird 30 min bei 0-5°C und weitere 1,5 h bei Zimmertemperatur gerührt. Nach Einengen unter vermindertem Druck bis zur Erzielung eines öligen Rückstands wird dieser in 15 ml Methanol und 5 ml Wasser aufgenommen, mit 2 n NaOH auf pH=8 eingestellt und weitere 1,5 h bei Zimmertemperatur gerührt. Methanol wird unter vermindertem Druck entfernt und die erhaltene wäßrige Lösung mit konzentrierter HCl auf pH=5 eingestellt. Die gelbe Lösung wird mit Chloroform extrahiert (1× 30 ml, 2× mit je 10 ml) und die CHCl₃-Phasen über MgSO₄ getrocknet. Nach Einengen der Lösung im vollen Membranpumpenvakuum werden 3 g eines gelben Öls erhalten. Nach Abziehen flüchtiger Bestandteile im vollen Membranpumpenvakuum ergibt eine Chromatographie an 60 g SiO₂ mit Chloroform:Methanol im Verhältnis 25:1 2,65 g Produkt als gelbes Öl. (DE-A-27 33 658)
- ¹H-NMR(CDCl₃): δ= 8,4(CHO, 0,5 H), 7,94 (CHO, 0,5H), 4,1 (quintett, 4H), 3,68 (t,2H), 1,7-2,19 (m,4H), 1,36(t,J=7 Hz, 6H)
- ¹³C-NMR(CDCL₃): δ= 162,65(CHO), 156,96(CHO), 61,72(OCH₂CH₃), 46,31(NCH₂, J=15,9 Hz), 22,15(PC, J=142,0 Hz), 19,13 (C-2) , 16,08(OCH₂CH₃)

### Beispiel 8

### Herstellung von 3-(N-Acetylhydroxyamino)-propylphosphonsäurediethylester

2,8 g (0,013 mol) 3-(N-Hydroxyamino)-propylphosphonsäurediethylester werden in 30 ml Methylenchlorid gelöst, und unter Eiskühlung werden 2,65 g (0,026 mol) Essigsäureanhydrid zugetropft. Die Reaktionsmischung wird 30 min lang bei 0-5°C und weitere 1,5 h bei Zimmertemperatur gerührt. Nach Einengen unter vermindertem Druck bis zur Erzielung eines gelben öligen Rückstands wurde in 15 ml Methanol und 5 ml Wasser aufgenommen, mit 2 n NaOH auf pH 8 eingestellt und weitere 1,5 h bei Zimmertemperatur gerührt. Methanol wird unter reduziertem Druck entfernt und die erhaltene Lösung mit konzentrierter HCl auf pH = 5 eingestellt. Die gelbe Lösung wird mehrmals mit Methylenchlorid extrahiert (1 × 30 ml, 2 × je 10 ml), die vereinigten CH₂Cl₂-Phasen werden über MgSO₄ getrocknet und das Lösungsmittel bei Zimmertemperatur unter vermindertem Druck entfernt. Es werden 3,7 g eines gelben Öls erhalten, das im vollen Membranpumpenvakuum von anhaftenden flüchtigen Substanzen befreit wird. Es bleiben 2,78 g gelbes Öl zurück.
- ¹³C-NMR(CDCL₃): δ= 171,96(C=O), 61,62(OCH₂CH₃), 47,44(J=15,49 Hz), 22,13(PC, J=141,8 Hz), 19,3, 15,9(OCH₂CH₃)

### Beispiel 9

### Herstellung von 3-(N-Formylhydroxyamino)-propylphosphonsäure-mono-Natriumsalz

Zu 4 ml Acetamid werden bei 0-5°C 2 ml Ameisensäure zugetropft. Es wird bei dieser Temperatur 10 min gerührt und weitere 15 min. bei Zimmertemperatur gerührt, anschließend wieder auf 0°C abgekühlt und 3,28 g (0,021 mol) 3-(N-Hydroxyamino)propylphosphonsäure, gelöst in 6 ml Ameisensäure bei 0-5°C zugetropft. Nach 1-stündigem Rühren bei Zimmertemperatur wird die Lösung im Rotationsverdampfer unter reduziertem Druck eingeengt, das Öl in 50 ml Methanol gelöst, auf 60°C erwärmt und mit 10 ml Ethanol versetzt. Ohne Rühren wird die entstehende ölige Ausscheidung durch Dekantieren abgetrennt. Die methanolische Lösung wird zum Ausfällen weißer Kristalle mit weiteren 50 ml Ethanol versetzt, aufgekocht und der weiße Rückstand abfiltriert. Dieser Rückstand wird in 80 ml Methanol aufgenommen und es werden unter Rühren 100 ml Ethanol hinzugefügt. Über Nacht wird bei Zimmertemperatur weitergerührt. Es wird ein Feststoff erhalten, der abfiltriert wird. (DE-A-27 33 658)

### Beispiel 10

### Herstellung von 3-(N-Acetylhydroxyamino)-propylphosphonsäure-mono-Natriumsalz

Eine Suspension von 3,8 g (0,02 mol) 3-(N-Hydroxyamino)-propylphosphonsäure wird in 20 ml Wasser vorgelegt, und es werden 4,51 g (0,044 mol) Essigsäureanhydrid bei Zimmertemperatur zugetropft. Nachdem 1,5 h lang bei Zimmertemperatur gerührt worden ist, wird mit 0,2 n NaOH ein pH-Wert von 2,5 eingestellt, die Lösung im vollen Membranpumpenvakuum eingeengt, zweimal in je 40 ml Wasser aufgenommen, die wieder durch Einengen entfernt werden, und das Öl zweimal mit je 30 ml Ether gewaschen, in 50 ml Wasser aufgenommen, und mit 1,6 g NaHCO₃ ein pH-Wert von 6,5 eingestellt. Nach Abziehen flüchtiger Bestandteile im Vakuum wird zum Entfernen des restlichen Wassers mit 20 ml n-Butanol versetzt, das ebenfalls unter reduziertem Druck entfernt wird. Das Öl wird zweimal mit Isopropanol ausgekocht, die Isopropanolphase verworfen und das zurückbleibende glasartige Harz mit einem Spatel zu einem gelblichen Feststoff (5,65 g) zerrieben. Zum Umkristallisieren wird in nur wenig Methanol aufgenommen, von ungelöstem filtriert und zum Filtrat tropfenweise Aceton zugegeben. Ein erstes Filtrieren ergibt 1 g Produkt mit einem Schmelzpunkt von 175°C. Zur weiteren Reinigung wird erneut wie oben beschrieben umkristallisiert. (DE-A-27 33 658)

### Beispiel 11

### Herstellung antiparasitär wirksamer Mittel

### Präparat für Injektionen

(1)Die erforderliche Menge des sterilen antiparasitär wirksamen Mittels, 3-(N-Formyl-N-hydroxylamino)-propylphosphonsäure-mononatriumsalz, wurde in Fläschchen oder Ampullen verteilt, die dann 500 mg Wirkstoff enthielten. Die Fläschchen wurden zum Ausschluß von Bakterien hermetisch abgeschlossen. Für Injektionen wurden jeweils 2 ml steriles Wasser zu den Fläschchen hinzugegeben, und der Inhalt wurde verabreicht.
   Im wesentlichen in gleicher Weise, wie vorstehend unter (1) beschrieben wurde, wurden weitere injizierbare Präparate der antiparasitär wirksamen Mittel, wie nachfolgend beschrieben, hergestellt:
(2)250 mg 3-(N-Formyl-N-hydroxylamino)-propylphosphonsäure-mononatriumsalz wurden als Wirkstoff für die Injektionen verwendet.
(3)250 mg 3-(N-Formyl-N-hydroxylamino)-trans-1-propenylphosphonsäure-mononatriumsalz wurden als Wirkstoff für die Injektionen verwendet.
(4)500 mg 3-(N-Acetyl-N-hydroxylamino)-2-hydroxypropylphosphonsäure-mononatriumsalz wurden als Wirkstoff für die Injektionen verwendet.
(5)250 mg 3-(N-Formyl-N-hydroxylamino)-2-hydroxypropylphosphonsäure-monokaliumsalz wurden als Wirkstoff für die Injektionen verwendet.

| Herstellung von Tabletten: | |
|---|---|
| Eine geeignete Tablettenrezeptur wird durch die folgende Mischung gebildet: | |
| 3-(N-Formyl-N-hydroxylamino)-propylphosphonsäure-mononatriumsalz | 200 mg |
| Mannit | 400 mg |
| Stärke | 50 mg |
| Magnesiumstearat | 10 mg |

| Herstellung von Kapseln | |
|---|---|
| 3-(N-Formyl-N-hydroxylamino)-propylphosphonsäüre-monokaliumsalz | 300 mg |
| Magnesiumstearat | 15 mg |

Die vorstehenden Bestandteile wurden vermischt und dann in eine harte Gelatinekapsel in herkömmlicher Weise eingebracht.

| | |
|---|---|
| Herstellung einer öligen Suspension 3-(N-Acetyl-N-hydroxylamino)-propylphosphonsäure-mononatriumsalz | 200 mg |
| Lanette-Wachs SX ® | 50 mg |
| Weiches Paraffin | 100 mg |
| Brilliant-blaü FCF | 25 mg |

Die obigen Bestandteile wurden mit flüssigem Paraffin für eine Gesamtmenge von 3 g vermischt unter Erzielung eines Infusionspräparates.

### Beispiele für Synthesewege für Stoffe mit der folgenden Struktur:

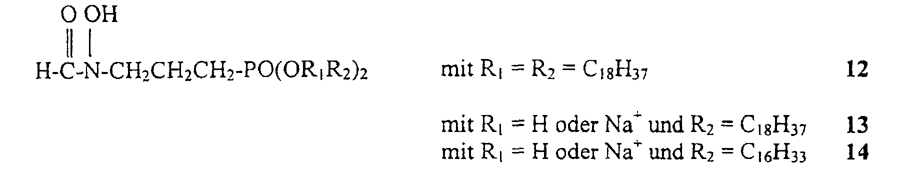

### Beispiel 12:

### 3-(N-Formyl-N-hydroxylamino)propylphosphonsäuredioctadecylester 12

1 Äquivalent Fosmidomycin (FR-31564) und 6 Äquivalente Tris(octadecyl)-orthoameisensäure werden unter reduziertem Druck und starkem Rühren 2 h unter Rückfluß erwärmt. Dann destilliert man - ebenfalls unter reduziertem Druck - Methanol sowie Ameisensäureoctadecylester ab, wobei die Temperatur unterhalb der Zersetzungstemperatur des Produktes gehalten werden muß. Im Ölpumpenvakuum werden weitere flüchtige Nebenprodukte entfernt, um schließlich **12** als hochviskoses Öl zu erhalten.
(Zur Durchführung vgl.: D.A. Nicholson, W.A. Cilley, O.T. Quimby, J Org Chem. 1970, 35, 3149-50)

Die Mono-Ester können sowohl ausgehend von Fosmidomycin als auch von Di-Octadecylester **12** dargestellt werden.

### Beispiel 13:

### 3-(N-Formyl-N-hydroxylamino)-propylphosphonsäure mono octadecylester 13

1. Vorschlag:
   0.21 mmol Fosmidomycin (Phosphonsäure) und 0.2 mmol n-Octadecanol werden in 1-2 ml trockenem Pyridin gelöst und 0.67 mmol Trichloracetonitril dazu getropft. Die Reaktionsmischung wird 16 h auf 80 °C erwärmt und dann im Vakuum eingeengt. Nach Aufnehmen in Wasser - zum Membranfiltrieren von Ungelöstem - wird erneut unter reduziertem Druck eingeengt und das Produkt an Silicagel mit Essigsäureethylester, Ethanol sowie Wasser als Laufmittel chromatographiert. Das Produkt **13** fällt dabei als zähe gummiartige bis glasartige Verbindung an.
   (Zur Durchführung vgl.: G.B.Brooks, D. Edwards, J.D.I. Hatto, T.C. Smale, R. Southgate, Tetrahedron 1995, 51, 7999-814)
2. Vorschlag:
   0.2 mmol des Diesters 3-(N-Formyl-N-hydroxylamino)-propylphosphonsäuredioctadecylester **13** gelöst in Ethanol wird mit 1 Äquivalent KOH (ethanolische Lösung) versetzt und 10 h unter Rückfluß gekocht. Durch Einleiten von CO₂ lassen sich Kaliumsalze als Carbonate fällen und durch Filtrieren entfernen. Das Filtrat engt man bis zur Trockene ein, trocknet das Öl über P₂O₅ , wäscht mit Petrolether und kann schließlich aus absolutem Ethanol durch Zugabe von Isopropanol das Produkt umkristallisieren.
   (Zur Durchführung vgl.: V. Jagodic, Chem Ber 1960, 93, 2308-13)

### Beispiel 14:

### 3-(N-Formyl-N-hydroxyamino)-propylphosphonsäure mono hexadecylester 14

**14** läßt sich analog zu **13** synthetisieren.

## Patentansprüche

1. Verwendung von phosphororganischen Verbindungen der allgemeinen Formel (I) 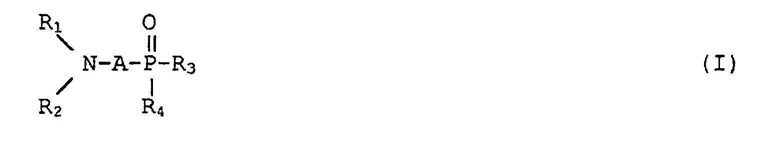 in der R₁ und R₂ gleich oder verschieden sind und aus der Gruppe ausgewählt sind, die aus Wasserstoff, substituiertem und unsubstituiertem Alkyl, substituiertem und unsubstituiertem Hydroxyalkyl, substituiertem und unsubstituiertem Alkenyl, substituiertem und unsubstituiertem Alkinyl, substituiertem und unsubstituiertem Aryl, substituiertem und unsubstituiertem Acyl, substituiertem und unsubstituiertem Cycloalkyl, substituiertem und unsubstituiertem Aralkyl, substituiertem und unsubstituiertem heterocyclischen Rest, Halogen, OX₁ und OX₂ besteht,
wobei X₁ und X₂ gleich oder verschieden sein können und aus der Gruppe ausgewählt sind, die aus Wasserstoff, substituiertem und unsubstituiertem Alkyl, substituiertem und unsubstituiertem Hydroxyalkyl, substituiertem und unsubstituiertem Alkenyl, substituiertem und unsubstituiertem Alkinyl, substituiertem und unsubstituiertem Aryl, substituiertem und unsubstituiertem Acyl, substituiertem und unsubstituiertem Cycloalkyl, substituiertem und unsubstituiertem Aralkyl, substituiertem und unsubstituiertem heterocyclischen Rest besteht,
A aus der Gruppe ausgewählt ist, die aus einem Alkylenrest, einem Alkenylenrest und einem Hydroxyalkylenrest besteht, R₃ und R₄ unabhängig aus der Gruppe ausgewählt sind, die aus Wasserstoff, substituiertem und unsubstituiertem C₁₋₂₆-Alkyl, substituiertem und unsubstituiertem Hydroxy-C₁₋₂₆-alkyl, substituiertem und unsubstituiertem Aryl, substituiertem und unsubstituiertem Acyl, substituiertem und unsubstituiertem Aralkyl, substituiertem und unsubstituiertem C₁₋₂₆-Alkenyl, substituiertem und unsubstituiertem C₁₋₂₆-Alkinyl, substituiertem und unsubstituiertem Cycloalkyl, substituiertem und unsubstituiertem heterocyclischem Rest, Halogen, OX₃ und OX₄ besteht,
wobei X₃ und X₄ unabhängig aus der Gruppe ausgewählt sind, die aus Wasserstoff, substituiertem und unsubstituiertem C₁₋₂₆-Alkyl, substituiertem und unsubstituiertem Hydroxyl-C₁₋₂₆-alkyl, substituiertem und unsubstituiertem Aryl, substituiertem und unsubstituiertem Aralkyl, substituiertem und unsubstituiertem C₁₋₂₆-Alkenyl, substituiertem und unsubstituiertem C₁₋₂₆-Alkinyl, substituiertem und unsubstituiertem Cycloalkyl, substituiertem und unsubstituiertem heterocyclischem Rest, einem Silyl, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, besteht,
und deren pharmazeutisch akzeptablen Salze, Ester und Salze der Ester
zur Herstellung eines Arzneimittels zur therapeutischen und prophylaktischen Behandlung von Infektionen bei Mensch und Tier, verursacht durch Parasiten, Pilze und Viren.

2. Verwendung nach Anspruch 1, **dadurch gekennzeichnet, daß** die phosphororganischen Verbindungen der Formel (II) 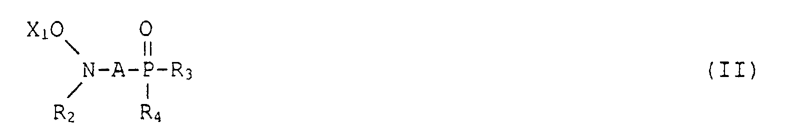 entsprechen, wobei
X₁ aus der Gruppe ausgewählt ist, die aus Wasserstoff, substituiertem oder unsubstituiertem Acyl, substituiertem oder unsubstituiertem Alkyl, substituiertem oder unsubstituiertem Aryl, substituiertem oder unsubstituiertem Aralkyl, substituiertem oder unsubstituiertem Cycloalkyl, substituiertem oder unsubstituiertem heterocyclischen Rest besteht.

3. Verwendung nach Anspruch 2,
**dadurch gekennzeichnet, daß**
R₂ ein Acylrest, bevorzugt ein Alkanoylrest und besonders bevorzugt Formyl- oder Acetylrest ist.

4. Verwendung nach einem der vorhergehenden Ansprüche,
**dadurch gekennzeichnet, daß**
R₃ und R₄ unabhängig aus der Gruppe ausgewählt sind, die aus Wasserstoff, Methyl, Ethyl besteht, oder R₄ für OX₄ steht, wobei X₄ aus der Gruppe ausgewählt ist, die aus Wasserstoff, Natrium, Kalium, Methyl und Ethyl besteht.

5. Verwendung nach einem der vorhergehenden Ansprüche, **dadurch gekennzeichnet, daß** A zwischen dem Phosphoratom und dem Stickstoffatom eine Kette aus drei Kohlenstoffatomen bildet, vorzugsweise eine Propylen-, Propenylen- oder Hydroxypropylenkette.

6. Verwendung nach Anspruch 1 oder 2,
**dadurch gekennzeichnet, daß**
R₄ für OX₄ steht und X₄ aus der Gruppe ausgewählt ist, die aus Wasserstoff, Ammonium und Metallen der ersten und zweiten Hauptgruppe des Periodensystems, vorzugsweise Natrium, Kalium, Calcium oder Magnesium, Ammoniumverbindungen, die sich von Ethylendiamin oder Aminosäuren ableiten, vorzugsweise Ethanolamin, Ethylendiamin, N,N-Dibenzylethylendiamin und Arginin, besteht.

7. Verwendung nach einem der vorhergehenden Ansprüche zur Behandlung von Infektionen, die durch Viren hervorgerufen werden, die aus der Gruppe ausgewählt sind, die aus Viren der Gattung Parvoviridae, insbesondere Parvoviren, Dependoviren, Densoviren, Viren der Gattung Adenoviridae, insbesondere Adenoviren, Mastadenoviren, Aviadenoviren, Viren der Gattung Papovaviridae, insbesondere Papovaviren, insbesondere Papillomaviren (sogenannte Warzenviren), Polyomaviren, insbesondere JC-Virus, BK-Virus, und Miopapovaviren, Viren der Gattung Herpesviridae, insbesondere Herpes-Simplex-Viren, der Varizellen/Zoster-Viren, menschlicher Zytomegalievirus, Epstein-Barr-Viren, humanes Herpesvirus 6, humanes Herpesvirus 7, humanes Herpesvirus 8, Viren der Gattung Poxviridae, insbesondere Pockenviren, Orthopox- Parapox-, Molluscum-Contagiosum-Virus, Aviviren, Capriviren, Leporipoxviren, primär hepatotropen Viren, insbesondere Hepatitisviren, wie Hepatitis-A-Viren, Hepatitis-B-Viren, Hepatitis-C-Viren, Hepatitis-D-Viren, Hepatitis-E-Viren, Hepatitis-F-Viren, Hepatits-G-Viren, Hepadnaviren, Viren der Gattung Picornaviridae, insbesondere Picornaviren, alle Enteroviren, alle Polioviren, alle Coxsackieviren, alle Echoviren, alle Rhinoviren, Aphthoviren, Viren der Gattung Calciviridae, Viren der Gattung Reoviridae, insbesondere Reoviren, Orbiviren, Rotaviren, Viren der Gattung Togaviridae, insbesondere Togaviren, Alphaviren, Rubiviren, Pestiviren, Rubellavirus, Viren der Gattung Flaviviridae, insbesondere Flaviviren, FSME-Virus, Viren der Gattung Orthomyxoviridae, insbesondere Influenzaviren, Viren der Gattung Paramyxoviridae, insbesondere Paramyxoviren, Morbillivirus, Pneumovirus, Masernvirus, Mumpsvirus, Viren der Gattung Rhabdoviridae, insbesondere Rhabdoviren, Rabiesvirus, Lyssavirus, viskuläres Stomatitisvirus, Viren der Gattung Coronaviridae, insbesondere Coronaviren, Viren der Gattung Bunyaviridae, insbesondere Bunyaviren, Nairovirus, Phlebovirus, Uukuvirus, Hantavirus, Hantaanvirus, Viren der Gattung Arenaviridae, insbesondere Arenaviren, lymphozytäres Choriomeningitis-Virus, Viren der Gattung Retroviridae, insbesondere Retroviren, alle HTL-Viren, humanes T-cell Leukämievirus, Oncornaviren, Spumaviren, Lentiviren, alle HI-Viren, Viren der Gattung Filoviridae, insbesondere Marburg- und Ebolavirus, Slow-Viren, Onkoviren und Leukämieviren besteht.

8. Verwendung nach einem der vorhergehenden Ansprüche zur Vorbeugung und Behandlung von Infektionen verursacht durch einzellige Parasiten, nämlich Erreger der Malaria, der Schlafkrankheit, der Chagas-Krankheit, der Toxoplasmose, der Amöbenruhr, der Leishmaniosen, der Trichomoniasis, der Pneumozystose, der Balantidiose, der Kryptosporidiose, der Sarkozystose, der Akanthamöbose, der Naeglerose, der Kokzidiose, der Giardiose und der Lambliose.

9. Verwendung nach einem der Ansprüche 1 bis 8 in einem pharmazeutischen Mittel, das einen wirksamen Gehalt an zumindest einer phosphororganischen Verbindung und einen pharmazeutisch akzeptablen Träger aufweist.

10. Verwendung nach Anspruch 9, **dadurch gekennzeichnet, daß** das pharmazeutische Mittel mindestens einen weiteren pharmazeutischen Wirkstoff, insbesondere Sulfonamid, Sulfadoxin, Artemisinin, Atovaquon, Chinin, Chloroquin, Hydroxychloroquin, Mefloquin, Halofantrin, Pyrimethamin, Armesin, Tetracycline, Doxycyclin, Proguanil, Metronidazol, Praziquantil, Niclosamid, Mebendazol, Pyrantel, Tiabendazol, Diethylcarbazin, Piperazin, Pyrivinum, Metrifonat, Oxamniquin, Bithionol oder Suramin aufweist.

11. Verwendung nach Anspruch 10, **gekennzeichnet durch** einen oder mehrere Bestandteile der Gruppe, die aus Penicillin, Benzylpenicillin (Penicillin G), Phenoxypenicillin, Isoxazolylpenicillin, Aminopenicillin, Ampicillin, Amoxixillin, Bacampicillin, Carboxypenicillin, Ticarcillin, Temocillin, Acyalaminopenicillin, Azlocillin, Mezlocillin, Piperacillin, Apalcillin, Mecillinam, Cephalosporin, der Cefazolin-Gruppe, der Cefuroxim-Gruppe, der Cefoxitin-Gruppe, Cefoxitin, Cefotetan, Cefmetazol, Latamoxef, Flomoxef, der Cefotaxim-Guppe, Cefozidim, der Ceftazidim-Gruppe, Ceftazidim, Cefpirom, Cefepim, den übrigen Cephalosporinen, Cefsulodin, Cefoperazon, Oralcephalosporinen der Cefalexin-Gruppe, Loracarbef, Cefprozil, neuen Oralcephalosporinen mit erweitertem Spektrum, Cefixim, Cefpodoxim-Proxetil, Cefuroxim-Axetil, Cefetamet, Cefotiam-Hexetil, Cefdinir, Ceftibuten, anderen β-Lactam-Antibiotika, Carbapenem, Imipenem/Cilastatin, Meropenem, Biapenem, Aztreonam, β-Lactamase-Hemmern, Clavulansäure/Amoxicillin, Clavulansäure/Ticarcillin, Sulbactam/Ampicillin, Tazobactam/Piperacillin, Tetracyclin, Oxytetracyclin, Rolitetracyclin, Doxycyclin, Minocyclin, Chloramphenicol, Aminoglykosiden, Gentamicin, Tobramycin, Netilmicin, Amikacin, Spectinomycin, Makrolid, Erythromycin, Clarithromycin, Roxithromycin, Azithromycin, Dirithromycin, Spiramycin, Josamycin, Lincosamid, Clindamycin, Fusidinsäure, Glykopeptid-Antibiotika, Vancomycin, Teicoplanin, Pristinamycin-Derivaten, Fosfomycin, antimikrobiellen Folsäureantagonisten, Sulfonamiden, Co-Trimoxazol, Trimethoprim, anderen Diaminopyrimidin-Sulfonamid-Kombinationen, Nitrofuranen, Nitrofurantoin, Nitrofurazon, Gyrase-Hemmern (Chinolone), Norfloxacin, Ciprofloxacin, Ofloxacin, Sparfloxacin, Enoxacin, Fleroxacin, Pefloxacin, Lomefloxacin, 1-Cyclopropyl-7-(2,8-diazabicyclo[4.3.0)]non-8-yl)-6-fluoro-8-chloro-1,4-dihydro-4-oxo-3-chinolincarbonsäurehydrochlorid (Bay Y3118), Nitroimidazolen, antimykobakteriellen Mitteln, Isoniazid, Rifampicin, Rifabutin, Ethambutol, Pyrazinamid, Streptomycin, Capreomycin, Prothionamid, Terizidon, Dapson, Clofazimin, Lokalantibiotika, Bacitracin, Tyrothricin, Polymyxine, Neomycin, Kanamycin, Paromomycin, Mupirocin, antiviralen Mitteln, Acyclovir, Ganciclovir, Azidothymidin, Didanosin, Zalcitabin, Thiacytidin, Stavudin, Ribavirin, Idoxuridin, Trifluridin, Foscarnet, Amantadin, Interferone, Tibol-Derivaten, Proteinase-Inhibitoren, Antimykotika, Polyenen, Amphothericin B, Nystatin, Natamycin, Azolen, Azolen zur septischen Therapie, Miconazol, Ketoconazol, Itraconazol, Fluconazol, Voriconazol (UK-109.496), Azolen für lokale Anwendung, Clotrimazol, Econazol, Isoconazol, Oxiconazol, Bifonazol, Flucytosin, Griseofulvin, Ciclopiroxolamin, Tolnaftat, Naftifin, Terbinafin, Amorolfin, Antrachinonen, Betulinsäure, Semianthrachinone, Xanthon, Naphtochinon, Arylaminoalkoholen, Chinin, Chinidin, Mefloquin, Halofantrin, Chloroquin, Amodiaquin, Acridin, Benzonaphthyridin, Mepacrin, Pyronaridin, Dapson, Sulfonamiden, Sulfadoxin, Sulfalene, Trimethoprim, Proguanil, Chlorproguanil, Diaminopyrimidine, Pyrimethamin, Primaquin, Aminochinoline, Tafenoquin(WR 238,605), Artemisinin, Dihydroartemisinin, 10b Artemether, Arteether, Artesunat, Atovaquon, Suramin, Melarsoprol, Nifurtmox, Stibogluconat-Natrium, Pentamidin, Amphotericin B, Metronidazol, Cliochinol, Mebendazol, Niclosamid, Praziquantel, Pyrantel, Tiabendazol, Diethylcarbamazin, Ivermectin, Bithionol, Oxamniquin, Metrifonat, Piperazin, Embonat besteht.

## Claims

1. Use of organophosphorus compounds of the general formula (I) 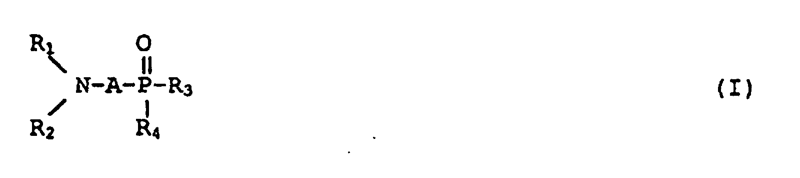 in which R₁ and R₂ are identical or different and are selected from the group which consists of hydrogen, substituted and unsubstituted alkyl, substituted and unsubstituted hydroxyalkyl, substituted and unsubstituted alkenyl, substituted and unsubstituted alkynyl, substituted and unsubstituted aryl, substituted and unsubstituted acyl, substituted and unsubstituted cycloalkyl, substituted and unsubstituted aralkyl, substituted and unsubstituted heterocyclic residue, halogen, OX₁ and OX₂, wherein X₁ and X₂ may be identical or different and are selected from the group which consists of hydrogen, substituted and unsubstituted alkyl, substituted and unsubstituted hydroxyalkyl, substituted and unsubstituted alkenyl, substituted and unsubstituted alkynyl, substituted and unsubstituted aryl, substituted and unsubstituted acyl, substituted and unsubstituted cycloalkyl, substituted and unsubstituted aralkyl, substituted and unsubstituted heterocyclic residue,
A is selected from the group which consists of an alkylene residue, an alkenylene residue and a hydroxyalkylene residue,
R₃ and R₄ are independently selected from the group which consists of hydrogen, substituted and unsubstituted C₁₋₂₆-alkyl, substituted and unsubstituted hydroxy-C₁₋₂₆-alkyl, substituted and unsubstituted aryl, substituted and unsubstituted acyl, substituted and unsubstituted aralkyl, substituted and unsubstituted C₁₋₂₆-alkenyl, substituted and unsubstituted C₁₋₂₆-alkynyl, substituted and unsubstituted cycloalkyl, substituted and unsubstituted heterocyclic residue, halogen, OX₃ and OX₄,
wherein X₃ and X₄ are independently selected from the group which consists of hydrogen, substituted and unsubstituted C₁₋₂₆-alkyl, substituted and unsubstituted hydroxyl-C₁₋₂₆-alkyl, substituted and unsubstituted aryl, substituted and unsubstituted aralkyl, substituted and unsubstituted C₁₋₂₆-alkenyl, substituted and unsubstituted C₁₋₂₆-alkynyl, substituted and unsubstituted cycloalkyl, substituted and unsubstituted heterocyclic residue, a silyl, a cation of an organic and inorganic base, in particular a metal from main groups I, II or III of the periodic system of elements, ammonium, substituted ammonium and ammonium compounds which are derived from ethylenediamine or amino acids,
and the pharmaceutically acceptable salts, esters and ester salts thereof
for the production of a pharmaceutical preparation for the therapeutic and prophylactic treatment of infections in humans and animals caused by parasites, fungi and viruses.

2. Use according to claim 1, **characterised in that** the organophosphorus compounds are of the formula (II) 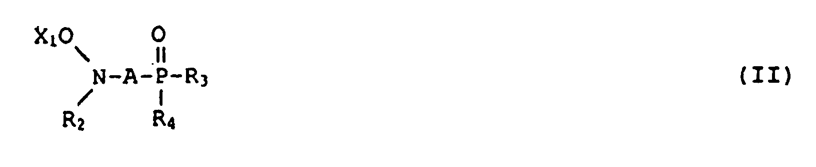 wherein
X₁ is selected from the group which consists of hydrogen, substituted or unsubstituted acyl, substituted or unsubstituted alkyl, substituted or unsubstituted aryl, substituted or unsubstituted aralkyl, substituted or unsubstituted cycloalkyl, substituted or unsubstituted heterocyclic residue.

3. Use according to claim 2,
**characterised in that**
R₂ is an acyl residue, preferably an alkanoyl residue and particularly preferably a formyl or acetyl residue.

4. Use according to one of the preceding claims,
**characterised in that**
R₃ and R₄ are independently selected from the group which consists of hydrogen, methyl, ethyl, or R₄ denotes OX₄, wherein X₄ is independently selected from the group which consists of hydrogen, sodium, potassium, methyl and ethyl.

5. Use according to one of the preceding claims,
**characterised in that**, between the phosphorus atom and the nitrogen atom, A forms a chain of three carbon atoms, preferably a propylene, propenylene or hydroxypropylene chain.

6. Use according to claim 1 or 2,
**characterised in that**
R₄ denotes OX₄ and X₄ is selected from the group which consists of hydrogen, ammonium and metals of main groups I and II of the periodic system, preferably sodium, potassium, calcium or magnesium, ammonium compounds derived from ethylenediamine or amino acids, preferably ethanolamine, ethylenediamine, N,N-dibenzylethylenediamine and arginine.

7. Use according to one of the preceding claims for the treatment of infections which are caused by viruses which are selected from the group which consists of viruses of the genus Parvoviridae, in particular parvoviruses, dependoviruses, densoviruses, viruses of the genus Adenoviridae, in particular adenoviruses, mastadenoviruses, aviadenoviruses, viruses of the genus Papovaviridae, in particular papovaviruses, in particular papillomaviruses ("wart" viruses), polyomaviruses, in particular JC virus, BK virus and miopapovaviruses, viruses of the genus Herpesviridae in particular herpes simplex viruses, varicella-zoster viruses, human cytomegalovirus, Epstein-Barr viruses, human herpesvirus 6, human herpesvirus 7, human herpesvirus 8, viruses of the genus Poxiviridae, in particular poxviruses, orthopoxviruses, parapoxviruses, molluscum contagiosum virus, aviviruses, capriviruses, leporipoxviruses, primarily hepatotropic viruses, in particular hepatitisviruses, such as hepatitis A viruses, hepatitis B viruses, hepatitis C viruses, hepatitis D viruses, hepatitis E viruses, hepatitis F viruses, hepatitis G viruses, hepadnaviruses, viruses of the genus Picornaviridae, in particular picornaviruses, all enteroviruses, all polioviruses, all coxsackie-viruses, all echoviruses, all rhinoviruses, aphthoviruses, viruses of the genus Calciviridae, viruses of the genus Reoviridae, in particular reoviruses, orbiviruses, rotaviruses, viruses of the genus Togaviridae, in particular togaviruses, alphaviruses, rubiviruses, pestiviruses, rubellavirus, viruses of the genus Flaviviridae, in particular flaviviruses, FSME virus, viruses of the genus Orthomyxoviridae, in particular influenza viruses, viruses of the genus Paramyxoviridae, in particular paramyxoviruses, morbillivirus, pneumovirus, measles virus, mumps virus, viruses of the genus Rhabdoviridae, in particular rhabdoviruses, rabies virus, lyssavirus, vascular stomatitisvirus, viruses of the genus Coronaviridae, in particular coronaviruses, viruses of the genus Bunyaviridae, in particular bunyaviruses, nairovirus, phlebovirus, uukuvirus, hantavirus, hantaan virus, viruses of the genus Arenaviridae, in particular arenaviruses, lymphocytic choriomeningitis virus, viruses of the genus Retroviridae, in particular retroviruses, all HTL viruses, human T-cell leukaemia virus, oncornaviruses, spumaviruses, lentiviruses, all HI viruses, viruses of the genus Filoviridae, in particular Marburg and Ebola virus, slow-viruses, oncoviruses and leukaemia viruses.

8. Use according to one of the preceding claims for the prevention and treatment of infections caused by unicellular parasites, namely the causative organisms of malaria, of sleeping sickness, of Chagas' disease, of toxoplasmosis, of amoebic dysentery, of leishmaniases, of trichomoniasis, of pneumocystosis, of balantidiasis, of cryptosporidiosis, of sarcocytosis, of acanthamoebosis, of naeglerosis, of coccidiosis, of giardiasis and of lambliasis.

9. Use according to one of claims 1 to 8 in a pharmaceutical agent which contains an active content of at least one organophosphorus compound and a pharmaceutically acceptable excipient.

10. Use according to claim 9, **characterised in that** the pharmaceutical agent comprises at least one further pharmaceutical active ingredient, in particular sulfonamide, sulfadoxine, artemisinin, atovaquone, quinine, chloroquine, hydroxychloroquine, mefloquine, halofantrine, pyrimethamine, armesin, tetracyclines, doxycycline, proguanil, metronidazole, praziquantel, niclosamide, mebendazole, pyrantel, tiabendazole, diethylcarbazine, piperazine, pyrivinium, metrifonate, oxamniquine, bithionol or suramin.

11. Use according to claim 10, **characterised by** one of more constituents from the group which consists of penicillin, benzylpenicillin (penicillin G), phenoxypenicillin, isoxazolylpenicillin, aminopenicillin, ampicillin, amoxicillin, bacampicillin, carboxypenicillin, ticarcillin, temocillin, acylaminopenicillin, azlocillin, mezlocillin, piperacillin, apalcillin, mecillinam, cephalosporin, the cefazolin group, the cefuroxime group, the cefoxitin group, cefoxitin, cefotetan, cefmetazole, latamoxef, flomoxef, the cefotaxime group, cefozidime, the ceftazidime group, ceftazidime, cefpirome, cefepime, other cephalosporins, cefsulodin, cefoperazone, oral cephalosporins of the cephalexin group, loracarbef, cefprozil, new broad-spectrum oral cephalosporins, cefixime, cefpodoxime-proxetil, cefuroxime-axetil, cefetamet, cefotiam-hexetil, cefdinir, ceftibuten, other β-lactam antibiotics, carbapenem, imipenem/cilastatin, meropenem, biapenem, aztreonam, β-lactamase inhibitors, clavulanic acid/amoxicillin, clavulanic acid/ticarcillin, sulbactam/ampicillin, tazobactam/piperacillin, tetracycline, oxytetracycline, rolitetracycline, doxycycline, minocycline, chloramphenicol, aminoglycosides, gentamicin, tobramycin, netilmicin, amikacin, spectinomycin, macrolide, erythromycin, clarithromycin, roxithromycin, azithromycin, dirithromycin, spiramycin, josamycin, lincosamide, clindamycin, fusidic acid, glycopeptide antibiotics, vancomycin, teicoplanin, pristinamycin derivatives, fosfomycin, antimicrobial folic acid antagonists, sulfonamides, co-trimoxazole, trimethoprim, other diaminopyrimidine-sulfonamide combinations, nitrofurans, nitrofurantoin, nitrofurazone, gyrase inhibitors (quinolones), norfloxacin, ciprofloxacin, ofloxacin, sparfloxacin, enoxacin, fleroxacin, pefloxacin, lomefloxacin, 1-cyclopropyl-7-(2,8-diazabicyclo[4.3.0])non-8-yl)-6-fluoro-8-chloro-1,4-dihydro-4-oxo-3-quinoline carboxylic acid hydrochloride (Bay Y3118), nitroimidazoles, antimycobacterial agents, isoniazid, rifampicin, rifabutin, ethambutol, pyrazinamide, streptomycin, capreomycin, prothionamide, terizidone, dapsone, clofazimine, topical antibiotics, bacitracin, tyrothricin, polymyxins, neomycin, kanamycin, paromomycin, mupirocin, antiviral agents, acyclovir, ganciclovir, azidothymidine, didanosine, zalcitabine, thiacytidine, stavudine, ribavirin, idoxuridine, trifluridine, foscarnet, amantadine, interferons, tibol derivatives, proteinase inhibitors, antimycotics, polyenes, amphotericin B, nystatin, natamycin, azoles, azoles for septic therapy, miconazole, ketoconazole, itraconazole, fluconazole, voriconazole (UK-109,496), azoles for topical use, clotrimazole, econazole, isoconazole, oxiconazole, bifonazole, flucytosine, griseofulvin, ciclopiroxolamine, tolnafnate, naftifine, terbinafine, amorolfine, anthraquinones, betulinic acid, semianthraquinones, xanthone, naphthoquinone, arylamino alcohols, quinine, quinidines, mefloquine, halofantrine, chloroquine, amodiaquine, acridine, benzonaphthyridine, mepacrine, pyronaridine, dapsone, sulfonamides, sulfadoxine, sulfalenes, trimethoprim, proguanil, chlorproguanil, diaminopyrimidines, pyrimethamine, primaquine, aminoquinolines, tafenoquine (WR 238,605), artemisinin, dihydroartemisinin, 10b artemether, arteether, atresunate, atovaquone, suramin, melarsoprol, nifurtimox, stibogluconate sodium, pentamidine, amphotericin B, metronidazole, clioquinol, mebendazole, niclosamide, praziquantel, pyrantel, tiabendazole, diethylcarbamazine, ivermectin, bithionol, oxamniquine, metrifonate, piperazine, embonate.

## Revendications

1. Utilisation de liaisons organophosphorées de formule générale (I) 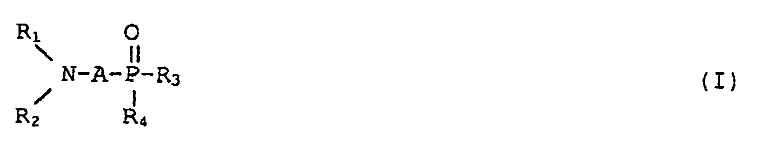 dans laquelle R₁ et R₂ sont identiques ou différents et sont choisis dans le groupe comprenant l'hydrogène, alkyle substitué et non substitué, hydroxyalkyle substitué et non substitué, alcényle substitué et non substitué, alcinyle substitué et non substitué, aryle substitué et non substitué, acyle substitué et non substitué, cycloalkyle substitué et non substitué, aralkyle substitué et non substitué, un reste hétérocyclique substitué et non substitué, halogène, OX₁ et OX₂,
dans lequel X₁ et X₂ peuvent être identiques ou différents et sont choisis dans le groupe comprenant l'hydrogène, alkyle substitué et non substitué, hydroxyalkyle substitué et non substitué, alcényle substitué et non substitué, alcinyle substitué et non substitué, aryle substitué et non substitué, acyle substitué et non substitué, cycloalkyle substitué et non substitué, aralkyle substitué et non substitué, un reste. hétérocyclique substitué et non substitué,
A est choisi dans le groupe comprenant un reste alkylène, un reste alcénylène et un reste hydroxyalkylène,
R₃ et R₄ sont choisis indépendamment dans le groupe comprenant l'hydrogène, alkyle en C₁₋₂₆ substitué et non substitué, hydroxy-alkyle-C₁₋₂₆ substitué et non substitué, aryle substitué et non substitué, acyle substitué et non substitué, aralkyle substitué et non substitué, alcényle en C₁₋₂₆ substitué et non substitué, alcinyle en C₁₋₂₆ substitué et non substitué, cycloalkyle substitué et non substitué, un reste hétérocyclique substitué et non substitué, un halogène, OX₃ et OX₄,
dans lequel X₃ et X₄ sont choisis indépendamment dans le groupe comprenant l'hydrogène, alkyle en C₁₋₂₆ substitué et non substitué, hydroxyl-alkyle-C₁₋₂₆ substitué et non substitué, aryle substitué et non substitué, aralkyle substitué et non substitué, alcényle en C₁₋₂₆ substitué et non substitué, alcinyle en C₁₋₂₆ substitué et non substitué, cycloalkyle substitué et non substitué, un reste hétérocyclique substitué et non substitué, un silyle, un cation d'une base organique et inorganique, en particulier un métal du premier, deuxième ou troisième groupe principal de la classification périodique, l'ammonium, l'ammonium et les groupements ammonium substitués, qui dérivent de l'éthylènediamine ou d'acides aminés,
et leurs sels, esters et sels d'ester pharmaceutiquement acceptables
pour la fabrication d'un médicament pour le traitement thérapeutique et prophylactique d'infections chez l'homme et l'animal, causées par des parasites, des champignons et des virus.

2. Utilisation selon la revendication 1, **caractérisée en ce que** les liaisons organophosphorées correspondent à la formule (II) 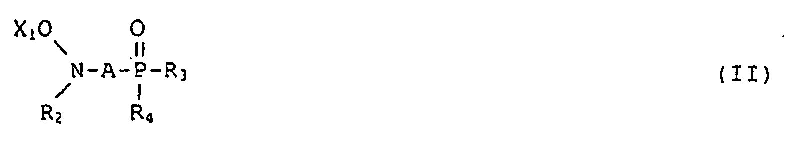 dans laquelle
X₁ est choisi dans le groupe comprenant l'hydrogène, acyle substitué ou non substitué, alkyle substitué ou non substitué, aryle substitué ou non substitué, aralkyle substitué ou non substitué, cycloalkyle substitué ou non substitué, un résidu hétérocyclique substitué ou non substitué.

3. Utilisation selon la revendication 2, **caractérisée en ce que**
R₂ est un reste acyle, de préférence un reste alkanoyle et de manière particulièrement préférée un reste formyle ou acétyle.

4. Utilisation selon l'une des revendications précédentes, **caractérisée en ce que**
R₃ et R₄ sont choisis indépendamment dans le groupe comprenant l'hydrogène, méthyle, éthyle ou R₄ correspond à OX₄, X₄ étant choisi dans le groupe comprenant l'hydrogène, le sodium, le potassium, méthyle et éthyle.

5. Utilisation selon l'une des revendications précédentes, **caractérisée en ce que** A forme entre l'atome de phosphore et l'atome d'azote une chaîne de trois atomes de carbone, de préférence une chaîne propylène, une chaîne propénylène ou une chaîne hydroxypropylène.

6. Utilisation selon la revendication 1 ou 2, **caractérisée en ce que**
R₄ correspond à OX₄ et X₄ est choisi dans le groupe comprenant l'hydrogène, l'ammonium ou des métaux du premier et du deuxième groupes principaux de la classification périodique, de préférence sodium, potassium, calcium ou magnésium, des groupements ammoniums qui dérivent de l'éthylènediamine ou d'acides aminés, de préférence éthanolamine, éthylènediamine, N,N-dibenzyléthylènediamine et arginine.

7. Utilisation selon l'une des revendications précédentes pour le traitement d'infections qui sont provoquées par des virus qui sont choisis dans le groupe comprenant les virus de l'espèce *Parvoviridae*, en particulier les parvovirus, les dépendovirus, les densovirus, les virus de l'espèce *Adenoviridae,* en particulier les adénovirus, les mastadénovirus, les aviadénovirus, les virus de l'espèce *Papovaviridae*, en particulier les papovavirus, en particulier les papillomavirus (aussi appelés virus de la verrue), les polyomavirus, en particuler le virus JC, le virus BK et les miopapovavirus, les virus de l'espèce *Herpesviridiae*, en particulier les virus herpès simplex, les virus varicella zoster, le cytomégalovirus humain, les virus d'Epstein-Barr, l'herpèsvirus humain 6, l'herpèsvirus humain 7, l'herpèsvirus humain 8, les virus de l'espèce *Poxviridae*, en particulier les virus de la variole, l'orthopoxvirus, le parapoxvirus, le virus Molluscum Contagiosum, les virus aviaires, les virus caprins, les léporipoxvirus, les virus hépatotropes primaires, en particulier les virus de l'hépatite comme les virus de l'hépatite A, les virus de l'hépatite B, les virus de l'hépatite C, les virus de l'hépatite D, les virus de l'hépatite E, les virus de l'hépatite F, les virus de l'hépatite G, les hepadnavirus, les virus de l'espèce *Picornaviridae*, en particulier les picornavirus, tous les entérovirus, tous les poliovirus, tous les virus coxsackie, tous les échovirus, tous les rhinovirus, les aphthovirus, les virus de l'espèce *Calciviridae*, les virus de l'espèce *Reoviridae*, en particulier les réovirus, les orbivirus, les rotavirus, les virus de l'espèce *Togaviridae*, en particulier les togavirus, les alphavirus, les rubivirus, les pestivirus, les rubellavirus, les virus de l'espèce *Flaviviridae*, en particulier les flavivirus, le virus TBE, les virus de l'espèce *Orthomyxoviridae*, en particulier les virus influenza, les virus de l'espèce *Paramyxoviridae*, en particulier les paramyxovirus, le morbillivirus, le pneumovirus, le virus de la rougeole, le virus des oreillons, les virus de l'espèce *Rhabdoviridae*, en particulier les rhabdovirus, le virus de la rage, le lyssavirus, le virus de la stomatite vésiculeuse, les virus de l'espèce *Coronaviridae*, en particulier les coronavirus, les virus de l'espèce *Bunyaviridae*, en particulier les bunyavirus, le nairovirus, le phlébovirus, l'uukuvirus, l'hantavirus, le virus Hantaan, les virus de l'espèce *Arenaviridae*, en particulier les arénavirus, le virus de la chorio-méningite lymphocytaire, les virus de l'espèce *Retroviridae*, en particulier les rétrovirus, tous les virus HTL, le virus de la leucémie humaine à lymphocytes T, les oncornavirus, les spumavirus, les lentivirus, tous les virus HI, les virus de l'espèce *Filoviridae*, en particulier le virus de Marburg et le virus Ebola, les « slow virus », les oncovirus et les virus de la leucémie.

8. Utilisation selon l'une des revendications précédentes pour la prévention et le traitement d'infections causées par des parasites monocellulaires, à savoir l'agent pathogène du paludisme, de la maladie du sommeil, de la maladie de Chagas, de la toxoplasmose, de la dysenterie amibienne, des leishmanioses, de la trichomoniase, de la pneumocystose, de la balantidiase, de la cryptosporidiose, de la sarcocystose, de l'acanthamoebose, de la naeglériase, de la coccidiose, de la giardiase et de la lambliase.

9. Utilisation selon l'une des revendications 1 à 8 dans un agent pharmaceutique qui présente une teneur efficace en au moins une liaison organophosphorée et un porteur pharmaceutiquement acceptable.

10. Utilisation selon la revendication 9, **caractérisée en ce que** l'agent pharmaceutique contient au moins une autre substance pharmaceutique active, en particulier sulfonamide, sulfadoxine, artémisinine, atovaquone, quinine, chloroquinine, hydroxychloroquine, méfloquine, halofantrine, pyriméthamine, armésine, tétracycline, doxycycline, proguanile, métronidazole, praziquantil, niclosamide, mébendazol, pyrantel, tiabendazol, diéthycarbazine, pipérazine, pyrivinum, métrifonate, oxamniquine, bithionol ou suramine.

11. Utilisation selon la revendication 10, **caractérisée par** un ou plusieurs éléments du groupe comprenant la pénicilline, la benzylpénicilline (pénicilline G), la phénoxypénicilline, l'isoxazolylpénicilline, l'aminopénicilline, l'ampicilline, l'amoxixilline, la bacampicilline, la carboxypénicilline, la ticarcilline, la témocilline, l'acyalaminopénicilline, l'azolicilline, la mezlocilline, la pipéraccilline, l'apalcilline, le mécillinam, la céphalosporine, du groupe céfazoline, du groupe céfuroxime, du groupe céfoxitine, la céfoxitine, le céfotétan, le cefmétazol, le latamoxef, le flomoxef, du groupe de la céfotaxime, la céfozidime, du groupe de la ceftazidime, la ceftazidime, le cefpirom, la céfépime, les anciennes céphalosporines, la cefsulodine, la céfopérazone, les céphalosporines orales du groupe de la céfalexine, le loracarbef, le cefprozil, les nouvelles céphalosporines orales à spectre élargi, la cefixime, la cefpodoxim-proxetil, la céfuroxim-axétil, le céfétamet, le céfotiam-hexétil, la cefdinir, le ceftibutène, d'autres antibiotiques du groupe des β-lactamines, le carbapénem, imipénem/cilastatine, le méropénem, le biapénem, l'aztréonam, les inhibiteurs de la β-lactamase, l'acide clavulanique/amoxicilline, l'acide clavulanique/ticarcilline, le sulbactam/ampicilline, le tazobactame/pipéracilline, la tétracycline, l'oxytétracycline, la rolitétracycline, la doxycycline, la minocycline, le chloramphénicol, les aminoglycosides, la gentamicine, la tobramycine, la nétilmicine, l'amikacine, la spectinomycine, les macrolides, l'érythromycine, la clarithromycine, la roxithromycine, l'azithromycine, la dirithromycine, la spiramycine, la josamycine, les lincosamides, la clindamycine, l'acide fusidique, les antibiotiques glycopeptidiques, la vancomycine, la téicoplanine, les dérivés de la pristinamycine, la fosfomycine, les antagonistes de l'acide folique antimicrobiens, les sulfonamides, le co-trimoxazol, le triméthoprim, d'autres combinaisons diaminopyrimidine-sulfonamide, les nitrofurans, la nitrofurantoïne, le nitrofurazone, les inhibiteurs de la gyrase (quinolone), la norfloxacine, la ciprofloxacine, l'ofloxacine, la sparfloxacine, l'énoxacine, la fléroxacine, la péfloxacine, la loméfloxacine, l'hydrochlorure d'acide 1-cyclopropyl-7-(2,8-diazabicyclo(4.3.0)]non-8-yl)-6-fluoro-8-chloro-1,4-dihydro-4-oxo-3-quinolinecarbonique (Bay Y3118), les nitroimidazoles, les agents antimycobactériens, l'isoniazide, la rifampicine, la rifabutine, l'éthambutol, le pyrazinamide, la streptomycine, la capréomycine, le prothionamide, le térizidon, le dapson, la clofazimine, les antibiotiques locaux, la bacitracine, la tyrothricine, la polymyxine, la néomycine, la kanamycine, la paromomycine, la mupirocine, des agents antiviraux, l'acyclovir, le ganciclovir, l'azidothymidine, la didanosine, la zalcitabine, la thiacytidine, la stavudine, la ribavirine, l'idoxuridine, la trifluridine, le foscarnet, l'amantadine, les interférons, les dérivés du tibol, les inhibiteurs de la protéinase, les antimycotiques, les polyènes, l'amphothéricine B, la nystatine, la natamycine, les azoles, les azoles pour traitement septique, le micanazole, le kétoconazole, l'itraconazole, le fluconazole, le voriconazole (UK-109.496), les azoles pour utilisation locale, le clotrimazole, l'éconazole, l'isoconazole, l'oxiconazole, le bifonazole, la flucytosine, la griséofulvine, la ciclopiroxolamine, le tolnaftate, la naftifine, la terbinafine, l'amorolfine, les antraquinones, l'acide bétulinique, la semianthraquinone, le xanthone, la naphtoquinone, l'arylaminoalcool, la quinine, la quinidine, la méfloquine, l'halofantrine, la chloroquine, l'amodiaquine, l'acridine, la benzonaphthyridine, la mépacrine, la pyronaridine, le dapson, les sulfonamides, la sulfadoxine, le sulfalène, le triméthoprim, le proguanil, le chlorproguanil, la diaminopyrimidine, la pyriméthamine, la primaquine, l'aminoquinoline, la tafénoquine (WR 238 605), l'artémisinine, la dihydroartémisinine, le 10b aréméther, l'artééther, l'artésunate, l'atovaquone, la suramine, le mélarsoprol, le nifurtmox, le stibogulconate sodique, la pentamidine, l'amphotéricine B, le métronidazol, le clioquinol, le mébendazole, le niclosamide, le praziquantel, le pyrantel, le tiabendazole, la diéthylcarbamazine, l'ivermectine, le bithionol, l'oxamniquine, le métrifonate, la pipérazine, l'embonate.
